(19) Europäisches Patentamt
European Patent Office
Office européen des brevets

(11) EP 3 172 191 B1

(12) # EUROPEAN PATENT SPECIFICATION

(45) Date of publication and mention of the grant of the patent:
**26.06.2019 Bulletin 2019/26**

(21) Application number: **15824692.6**

(22) Date of filing: **22.07.2015**

(51) Int Cl.:
***C07H 17/04*** (2006.01) ***A61K 31/7048*** (2006.01)
***A61K 31/357*** (2006.01) ***A61K 36/80*** (2006.01)
***A61P 11/06*** (2006.01) ***A61P 29/00*** (2006.01)

(86) International application number:
**PCT/KR2015/007647**

(87) International publication number:
**WO 2016/013877 (28.01.2016 Gazette 2016/04)**

(54) **A NOVEL COMPOUND ISOLATED FROM PSEUDOLYSIMACHION ROTUNDUM VAR. SUBINTEGRUM CONTAINING ABUNDANT AMOUNT OF ACTIVE INGREDIENT, THE COMPOSITION COMPRISING THE SAME FOR PREVENTING OR TREATING ALLERGY DISEASE, INFLAMMATORY DISEASE, ASTHMA OR CHRONIC OBSTRUCTIVE PULMONARY DISEASE AND THE USE THEREOF**

NEUARTIGE, AUS PSEUDOLYSIMACHION ROTUNDUM VAR. ISOLIERTE VERBINDUNG SUBINTEGRUM MIT HOHEM GEHALT EINES WIRKSTOFFS, ZUSAMMENSETZUNG DAMIT ZUR PRÄVENTION ODER BEHANDLUNG EINER ALLERGIEKRANKHEIT, ENTZÜNDUNGSKRANKHEITEN, ASTHMA ODER CHRONISCH OBSTRUKTIVER LUNGENERKRANKUNG UND VERWENDUNG DAVON

NOUVEAU COMPOSÉ ISOLÉ À PARTIR DE PSEUDOLYSIMACHION ROTUNDUM VAR. SUBINTEGRUM CONTENANT UNE QUANTITÉ ABONDANTE D'INGRÉDIENT ACTIF, COMPOSITION LE COMPRENANT POUR LA PRÉVENTION OU LE TRAITEMENT D'UNE MALADIE ALLERGIQUE, D'UNE MALADIE INFLAMMATOIRE, DE L'ASTHME OU D'UNE MALADIE PULMONAIRE OBSTRUCTIVE CHRONIQUE ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.07.2014 KR 20140094023**

(43) Date of publication of application:
**31.05.2017 Bulletin 2017/22**

(73) Proprietors:
- **Yungjin Pharmaceutical Co., Ltd.**
  **Gangdong-gu**
  **Seoul 134-721 (KR)**
- **Korea Research Institute of Bioscience and Biotechnology**
  **Daejeon 305-806 (KR)**

(72) Inventors:
- **LEE, Yongnam**
  **Suwon-si**
  **Gyeonggi-do 443-280 (KR)**
- **YOO, Ji-seok**
  **Suwon-si**
  **Gyeonggi-do 441-841 (KR)**
- **SHIN, Dae-hee**
  **Seoul 137-860 (KR)**
- **RYOO, Byung-hwan**
  **Seongnam-si**
  **Gyeonggi-do (13633) (KR)**
- **OH, Sei-Ryang**
  **Daejeon 305-806 (KR)**
- **AHN, Kyung-Seop**
  **Daejeon 305-806 (KR)**
- **LEE, Hyeong-Kyu**
  **Daejeon 305-806 (KR)**
- **LEE, Su Ui**
  **Daejeon 305-806 (KR)**
- **SONG, Hyuk-Hwan**
  **Sungdong-gu**
  **Seoul (04724) (KR)**
- **SHIN, In-Sik**
  **Yongbong-ro, Buk-gu**
  **Gwangju (61186) (KR)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **RYU, Hyung Won**
**Daejeon 305-806 (KR)**

(74) Representative: **Grünecker Patent- und Rechtsanwälte**
**PartG mbB**
**Leopoldstraße 4**
**80802 München (DE)**

(56) References cited:

| | |
|---|---|
| **WO-A1-2006/129964** | **WO-A1-2014/104672** |
| **KR-A- 20060 125 489** | **KR-A- 20060 125 499** |
| **KR-A- 20090 117 174** | **KR-A- 20140 087 982** |

- **SØREN R. JENSEN ET AL: "Chlorinated Iridoid Glucosides from Veronica longifolia and Their Antioxidant Activity", JOURNAL OF NATURAL PRODUCTS., vol. 73, no. 9, 24 September 2010 (2010-09-24), pages 1593-1596, XP055427325, US ISSN: 0163-3864, DOI: 10.1021/np100366k**
- **SONG HYUK-HWAN ET AL: "Piscroside C, a novel iridoid glycoside isolated from Pseudolysimachion rotundum var. subintegrum suppresses airway inflammation induced by cigarette smoke", JOURNAL OF ETHNOPHARMACOLOGY, vol. 170, 30 April 2015 (2015-04-30), pages 20-27, XP029174758, ISSN: 0378-8741, DOI: 10.1016/J.JEP.2015.04.043**
- **JIA, QI ET AL.: 'Pikuroside: A Novel Iridoid from Picrorhiza kurroa' J. NAT. PROD. vol. 62, no. 6, 01 January 1999, pages 901 - 903, XP002504846 DOI: 10.1021/NP980493**

SEQUENCE LISTING

<110> Yungjin Pharmaceutical Co., Ltd. Korea Research Institute of Bioscience and Biotechnology

<120> A NOVEL COMPOUND ISOLATED FROM PSEUDOLYSIMACHION ROTUNDUM VAR. SUBINTEGRUM CONTAINING ABUNDANT AMOUNT OF ACTIVE INGREDIENT, THE COMPOSITION COMPRISING THE SAME FOR PREVENTING OR TREATING ALLERGY DISEASE, INFLAMMATORY DISEASE, ASTHMA OR CHRONIC OBSTRUCTIV

<130> EP110074FZSZpau

<140> 15824692.6

<141> 2015-07-22

<150> PCT/KR2015/007647
<151> 2015-07-22

<150> 10-2014-0094023
<151> 2014-07-24

<160> 4

<170> PatentIn version 3.5

<210> 1
<211> 21
<212> DNA
<213> Homo Sapiens

<220>
<223> Mucin 5AC (MUC5AC)

<400> 1
tgatcatcca gcagcagggc t 21

<210> 2
<211> 22
<212> DNA
<213> Homo Sapiens

<220>
<223> Mucin 5AC (MUC5AC)

<400> 2
ccgagctcag aggacatatg gg 22

<210> 3
<211> 24
<212> DNA
<213> Homo Sapiens

<220>
<223> Glyceraldehyde-3-Phosphate Dehydrogenase (GAPDH)

<400> 3
cggagtcaac ggatttggtc gtat 24

<210> 4
<211> 24
<212> DNA
<213> Homo Sapiens

<220>
<223> Glyceraldehyde-3-Phosphate Dehydrogenase (GAPDH)

<400> 4
agccttctcc atggtggtga agac 24

## Description

### Technical Field

The present invention relates to a novel compound isolated from *Pseudolysimachion* rotundum var. subintegrum, the composition comprising the same preventing or treating allergic disease, inflammatory disease, asthma or chronic obstructive pulmonary disease and the use thereof.

### Background Art

Generally, an inflammatory response is a normal response of human body associated with an edema, a pain etc in case that a tissue or a cell received any invasion causing some organic change in the tissue or cell. Recently, various kinds of cytokines have been found to be involved in the inflammatory disease.

Allergic reaction may be classified into four categories, i.e., type I, II, III and IV according to the types of response or two categories, i.e., immediate type allergic reaction such as type I, II or III, and delayed type allergic reaction such as type IV according to the types of the period from the re-sensitization time caused by allergen to the onset time of reaction.

Among them, type I allergy, being involved in IgE antibody and called as anaphylaxis type allergy, causes to a bronchial asthma, atopic diseases such as dermatitis or gastroenteritis etc, allergic rhinitis such as pollenosis, allergic conjunctivitis, food allergy and the like.

Asthma is regarded as a complex syndrome of the airways that is characterized by various clinical symptoms, for example, cough, dyspnea caused by airflow obstruction, acute or chronic airway inflammation, airway hyperresponsiveness(AHR) and structural remodeling and can be reversibly or irreversibly recoverable. Most of asthma is allergic disease and is characterized by chronic airway inflammation and bronchial hyperresponsiveness (Minoguchi K and Adachi M., Pathophysiology of asthma. In: Cherniack NS, Altose MD, Homma I. editors. Rehabilitation of the patient with respiratory disease. New York: McGraw-Hill, 1999, pp97-104).

The asthma can be classified two types, i.e., extrinsic asthma and intrinsic asthma. The extrinsic asthma is caused by exposing antigen and it is shown positive reaction in skin test or bronchial provocation test against the antigen. Usually causing ages is getting younger. It is mainly caused by House Dust Mite Dermatophagoides and pollen, epithelium of animal, fungi and so on. The intrinsic asthma is caused by upper respiratory infections, exercise, emotional instability, changing of climate of humidity and it is common to adult patient. Also, the IgE antigen of extrinsic asthma can be detected by skin test due to increasing IgE in serum.

With regards to pathophysiology, asthma is recognized by T-helper2 (Th2)-cell-driven chronic inflammation, and a variety of inflammatory mediators, such as cytokines, chemokines, signaling molecules, adhesion molecules and growth factors, from immune cells and structural cells in the airways are involved in various stages of asthma (Elias JA et al., J Clin Invest.2003, 111, pp291-297). The activated inflammatory cells such as eosinophil, mast cells, alveolar macrophage etc in the bronchus of patients suffering from asthma, release various inflammatory mediators such as cystein leukotrienes, prostaglandins etc and is involved in potent bronchial constriction (Maggi E. et al., Immunotechnology 1998, 3, 233-244.; Pawankar R. et al., Curr. Opin. Allergy Clin. Immunol.2001, 1, 3-6.; Barnes PJ et al., Phamacol. Rev.1998 , 50, 515-596).

Accordingly, since the reproduction of various cytokines involved in inflammatory cell activation, such as IL-4, IL-5, IL-13 etc and IgE and reproduction of cystein leukotrienes released from the inflammatory cells are the main causes of inflammation, allergic reaction and asthma, there have been much studied to develop the inhibiting agents from the reproduction of those till now.

Generally, chronic obstructive pulmonary disease (COPD) is one of pulmonary disease caused by abnormal inflammatory disease in lung resulting in the obstruction of respiratory tract. COPD gives rise to dyspnoea resulting from the hindrance from exhausting air flow and shows different characteristics for example, the poor reversibility of an airways limitation or airways obstruction, the progressive development according to elapse time etc, from the common characteristics of asthma and may be classified into a pulmonary emphysema and chronic obstructive bronchitis (Barnes P.J., Pharmacol.Rev. 2004, 56, 515-548).

COPD has been reported as one of risk factor for cardiovascular morbidity and mortality and the fifth leading cause of death worldwide in 2001. The prevalence of chronic obstructive pulmonary disease based on Global Initiative for Chronic Obstructive Lung Disease (GOLD) criteria (a ratio of FEV1 to FVC of less than 0.7) was 17.2% (men, 25.8%; women, 9.6%) among Koreans older than 45 years (Kim, D. S. et al., Am. J. Respir. Crit. Care Med.2005, 172, 842-847.; Sin, D.D. et al., Proc. Am. Thorac. Soc.2005, 2, 8-11.; Buist A.S.et al.,Lancet, 2007, 370, 741-750).

Most patients with COPD have all three pathological mechanisms (chronic obstructive bronchitis, emphysema, and mucus plugging) as all are induced by smoking, but they may differ in the proportion of emphysema and obstructive bronchitis. In developed countries, cigarette smoking is by far the most common cause of COPD, but there are several other risk factors, including air pollution (particularly, indoor air pollution from burning fuels), poor diet, and occupational

exposure. COPD is characterized by acceleration in the normal decline of lung function seen with age. The slowly progressive airflow limitation leads to disability and premature death and is quite different from the variable airway obstruction and symptoms in asthma, which rarely progresses in severity.

There have been reported that the pathophysiological action and syndrome of COPD are fundamentally different from those of asthma. Although COPD and asthma both involve inflammation in the respiratory tract, there are marked differences in the nature of the inflammatory process, with differences in inflammatory cells, mediators, response to inflammation, anatomical distribution, and response to anti-inflammatory therapy, for example, (a) in respect to inflammatory cells, mast cell, eosinophils, $CD4^+$ cell (Th2), macrophages etc mainly act on the occurrence of asthma whereas neutrophils, $CD8^+$ (Tc) etc mainly act on the occurrence of COPD; (b) in respect to inflammatory mediators, leukotriens B, histamine, IL-4, IL-5, IL-13, eotaxin, RENTES, oxidative stress etc are mainly involved in the occurrence of asthma whereas TNF-alpha, IL-8, GRO-alpha etc are mainly involved in the occurrence of COPD; (c) in respect to inflammatory syndrome, asthma shows different inflammatory syndrome by acting on the overall pulmonary tract at early age, such as AHR (airway hyperresponsiveness), epithelial shedding, fibrosis, no parenchymal involvment, muscus secretion, relatively reversible airways obstruction, cough, sneezing, dyspnea etc from that of COPD, which occurs by acting on peripheral airways at adults and shows various phenomena such as, epithelial metaplasia, parenchymal destruction, relatively irreversible airways obstruction, chronic bronchitis, emphysema etc (Barnes PJ., Chest 2000, 117, 10S-14S.; Saetta M. et al., Am. J. Respir. Crit. CareMed. 2001, 163, 1304-1309).

Histopathological studies on COPD show a predominant involvement of peripheral airways (bronchioles) and lung parenchyma, whereas asthma involves inflammation in all airways but without involvement of the lung parenchyma. There is obstruction of bronchioles, with fibrosis and infiltration with macrophages and T lymphocytes. There is destruction of lung parenchyma, as well as an increased number of macrophages and CD8 (cytotoxic) T lymphocytes (Saetta M. et al., Am. J. Respir. Crit. Care Med. 1998, 157, 822-826). Bronchial biopsies show similar changes with an infiltration of macrophages and CD8 cells and an increased number of neutrophils in patients with severe COPD (Di Stefano A. et al., Am. J. Respir. Crit. Care Med. 1998, 158, 1277 -1285).

In contrast to asthma, eosinophils are not prominent except during exacerbations or when patients have concomitant asthma (Fabbri L. et al., Thorax 1998, 53, 803-808.; Fabbri LM. et al., Am. J. Respir. Crit. Care Med. 2003, 167, 418-424).

Accordingly, the therapeutic approach of chronic obstructive pulmonary disease (COPD) shall be different from that of asthma; however, the present therapy has been focused on treating non-specifically both of diseases. Therefore, there have been no anti-inflammatory therapies specifically approved for COPD and the available anti-inflammatory therapies were originally developed for asthma. The challenges facing research in COPD are multi-faceted; the mechanisms underlying the complex and heterogeneous pathology of this disease require unravelling; the role of inflammation in disease progression needs to be confirmed. (Hele D. et al., Expert. Opino. Invest. Drug , 2003, 12, 5-18.; Fox, J C. et al., Curr. Opin. Pharmacol. 2009, 9, 231-242).

Improvements to the current therapy available to treat asthma in the form of longer acting beta-agonists, safer steroids and combination therapies are ongoing and for COPD anti-cholinergics provide symptomatic relief. Steroids have been used to treat exacerbations, but as yet, no treatment has been shown to impact significantly on the progressive decline in lung function in COPD or the development of asthma.

Accordingly, there have been much studied to develop new drugs with potential to successfully and specifically treat allergic disease, inflammatory disease, asthma or COPD till now.

The present inventors have been focused to develop potent treating agent derived from natural resources with safety and efficacy such as plant, animals etc having potent treating activity of allergic disease, inflammatory disease, asthma or COPD and finally, have subsequently found unexpectedly surprising results, for example, potent anti-inflammatory, anti-allergy and anti-asthma activity of the extract of *Pseudolysimachion* longifolium (Korean Patent No. 10-860080 B1 and US 2012/0183632 A1) and various compounds isolated therefrom such as, verproside (6-*O*-3,4-dihydroxybenzoyl catalpol), picroside II (6-*O*-4-hydroxy-3-methoxybenzoyl catalpol), verminoside (6-*O*-3,4-Dihydroxy cinnamoyl catalpol), 6-*O*-veratroyl catalpol (6-*O*-3,4-Dimethoxy benzoyl catalpol), minecoside (6-*O*-3-hydroxy-4-methoxycinnamoyl catalpol), catalpol and the like (Korean Patent Publication No. 10-2006-125499 A1; WO 2006/129964 A1; WO 2014/104672 A1); potent anti-inflammatory, anti-allergy and anti-asthma activity of the novel purified extract containing abundant active ingredients such as catalpol derivatives from the extract of *Pseudolysimachion* rotundum var subintegrum (ATC2: Korean Patent No. 1476045 B1, ATC1: Korean Patent No. 1504651 B1); and anti-COPD activity of those extract (Korean Patent No. 1476095 B1) till now.

*Pseudolysimachion* rotundum var subintegrum, is a perennial herb distributed in Korea, China, Japan, Ostrov Sakhalin, and Russia.

Based on the previous studies on the anti-inflammatory, anti-allergy and anti-asthma activity of the extract of *Pseudolysimachion* rotundum var subintegrum, the present inventors have tried to develop novel compound showing anti-inflammatory, anti-allergy, anti-asthma and anti-COPD activity isolated from the extract of *Pseudolysimachion* rotundum var subintegrum.

However, there has been not reported or disclosed on novel compound showing anti-inflammatory, anti-allergy, anti-asthma and anti-COPD activity isolated from the extract of *Pseudolysimachion* rotundum var subintegrum in the above cited literatures, the disclosures of which are incorporated herein by reference.

Accordingly, the present inventors have found novel compound showing anti-inflammatory, anti-allergy, anti-asthma and anti-COPD activity isolated from the extract of *Pseudolysimachion* rotundum var subintegrum and the inventive compound showed potent anti-inflammatory, anti-allergy, anti-asthma and anti-COPD activity through various *in vitro* tests, for example, (1) cyto-toxicity test using by HT1080, H292 and EL4 cell line, (2) an inhibition test on the expression of MUC5AC (oligomeric muscus/gel-forming) induced by TNF-alpha, (3) NF-kappa B luciferase reporter assay, (4) inhibition test on the activity of NF-kappa B transcription factor; (5) inhibition on mRNA expression of target genes such as MMP-9, MUC5AC and IL-4 by way of inhibiting the NF-kappa B activity; (6) dose-dependent inhibiting effect on MUC5AC reproduction through MUC5AC protein reproduction assay; as well as in vivo tests, for example, (7) reducing effect on the number of inflammatory cells such as eosiniphil using by OVA-sensitized/challenged mouse model, (8) inhibition test on the release of IgE, inflammatory cytokines such as IL-4, IL-5, IL-13 etc in BALF fluid and infiltration of inflamed cells, as well as the suppression of airway hyperresponsiveness and golblet cell hyperplasia, (9) an inhibition test using by COPD animal model (C57B/6N mouse) on the proliferation of inflammatory cells in BALF fluid, the inhibition on the reproduction of ROS (Reactive oxygen species), and activity of neutrophil elastase, the decreasing effect on the level of IL-6, TNF-alpha and the infiltrated inflammatory cells etc.

## Disclosure of Invention

### Technical Problem

The present invention provides a novel compound (KS534) isolated from the extract of *Pseudolysimachion* rotundum var subintegrum.

The present invention also provides a pharmaceutical composition and a health food comprising the novel compound (KS534) isolated from the extract of *Pseudolysimachion* rotundum var subintegrum as an active ingredient in an effective amount for use in treating and preventing allergic disease, inflammatory disease, asthma or COPD disease.

The present invention also provides a use of the novel compound (KS534) isolated from the extract of *Pseudolysimachion* rotundum var subintegrum for manufacture of medicines employed for treating or preventing inflammatory disease, allergic disease, asthma or COPD.

The present invention also provides the novel compound (KS534) for use in a method of treating or preventing inflammatory disease, allergic disease, asthma or COPD in a mammal or human comprising administering to said mammal or human an effective amount of the novel compound (KS534) isolated from the extract of *Pseudolysimachion* rotundum var subintegrum, together with a pharmaceutically acceptable carrier thereof.

### Solution to Problem

The present invention provides (3R,5S,5aS,6R,7S,8R,8aS)-8-chloro-8a-hydroxy-5 -(((2S,3R,4S,5S,6R)-3,4,5-trihydroxy-6 (hydroxymethyl)tetrahydro-2H-pyran-2-yl)oxy)-1H-3,6-methanocyclopenta[e][1,3]dioxepin-7-yl 3,4-dihydroxybenzoate (KS534) represented by the following chemical formula (1), the pharmaceutically acceptable salt or solvates thereof:

[Chemical Formula 1]

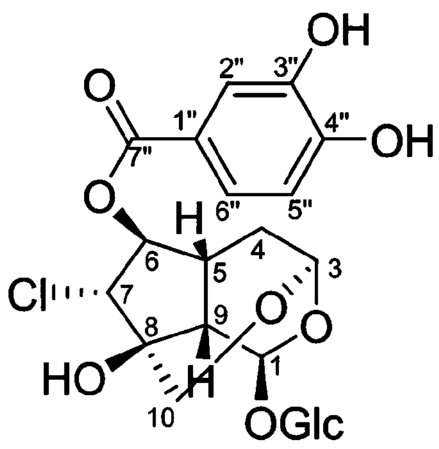

(1)

The inventive novel compound can be transformed into their pharmaceutically acceptable salt and solvates by the conventional method well known in the art. For the salts, acid-addition salt thereof formed by a pharmaceutically acceptable free acid thereof is useful and can be prepared by the conventional method. For example, after dissolving the compound in the excess amount of acid solution, the salts are precipitated by the water-miscible organic solvent such as methanol, ethanol, acetone or acetonitrile to prepare acid addition salt thereof and further the mixture of equivalent amount of compound and diluted acid with water or alcohol such as glycol monomethylether, can be heated and subsequently dried by evaporation or filtrated under reduced pressure to obtain dried salt form thereof.

As a free acid of above-described method, organic acid or inorganic acid can be used. For example, organic acid such as methansulfonic acid, *p*-toluensulfonic acid, acetic acid, trifluoroacetic acid, citric acid, maleic acid, succinic acid, oxalic acid, benzoic acid, lactic acid, glycolic acid, gluconic acid, galacturonic acid, glutamic acid, glutaric acid, glucuronic acid, aspartic acid, ascorbic acid, carbonylic acid, vanillic acid, hydroiodic acid and the like, and inorganic acid such as hydrochloric acid, phosphoric acid, sulfuric acid, nitric acid, tartaric acid and the like can be used herein.

Further, the pharmaceutically acceptable metal salt form of inventive compounds may be prepared by using base. The alkali metal or alkali-earth metal salt thereof can be prepared by the conventional method, for example, after dissolving the compound in the excess amount of alkali metal hydroxide or alkali-earth metal hydroxide solution, the insoluble salts are filtered and remaining filtrate is subjected to evaporation and drying to obtain the metal salt thereof. As a metal salt of the present invention, sodium, potassium or calcium salt are pharmaceutically suitable and the corresponding silver salt can be prepared by reacting alkali metal salt or alkali-earth metal salt with suitable silver salt such as silver nitrate.

The pharmaceutically acceptable salt of the compound comprise all the acidic or basic salt which may be present at the compounds, if it does not indicated specifically herein. For example, the pharmaceutically acceptable salt of the present invention comprise the salt of hydroxyl group such as the sodium, calcium and potassium salt thereof; the salt of amino group such as the hydrogen bromide salt, sulfuric acid salt, hydrogen sulfuric acid salt, phosphate salt, hydrogen phosphate salt, dihydrophosphate salt, acetate salt, succinate salt, citrate salt, tartarate salt, lactate salt, mandelate salt, methanesulfonate (mesylate) salt and *p*-toluenesulfonate (tosylate) salt etc, which can be prepared by the conventional method well known in the art.

There may exist in the form of optically different diastereomers since the disclosed compounds have unsymmetrical centers, accordingly, the compounds of the present disclosure comprise all the optically active isomers, R or S stereoisomers and the mixtures thereof. The application further discloses all the uses of racemic mixture, more than one optically active isomer or the mixtures thereof as well as all the preparation or isolation method of the diastereomer well known in the art.

The compounds of the invention may be isolated from the isolation or purification method which is well known in the art such as Silica gel column chromatography or re-crystalization method, for example, the method disclosed in Korea Patent Publication No. 10-2006-125499; or chemically synthesized by the well-known methods in the art, which are merely exemplary and in no way limit the invention.

The inventive novel compound (KS 534) may be prepared from *Pseuclolysimachion rotundum* var *subintegrum* by following procedure:

For example, novel compound (KS 534) is characterized by being prepared by the process of; adding at least one extracting solvent selected from water, C1-C4 lower alcohol such as methanol, ethanol, butanol etc or the mixtures thereof, preferably, mixture of water and methanol, more preferably, 10 - 100 % (w/w) methanol in water to dried *Pseu-*

*dolysimachion* rotundum var *subintegrum* at the 1st step; subjecting to at least one extraction method selected from reflux extraction with hot water, cold water extraction, ultra-sonication or conventional extraction, preferably cold water extraction at the temperature ranging from 10 to 150°C, preferably from 20 to 70°C, for the period ranging from 30 mins to 72 hours, preferably, 1 to 48 hours, more preferably, cold water extraction at the temperature ranging from 10 to 60°C, preferably from 20 to 50°C, for the period ranging from 30 mins to 72 hours, preferably, 6 to 48 hours repeatedly, and then reflux extraction at the temperature ranging from 40 to 120°C, preferably from 60 to 90°C, for the period ranging from 30 mins to 72 hours, preferably, 6 to 48 hours, repeatedly, to afford the 1st extract at 2nd step; subjecting to filtration, concentration under vaccuo and drying method to afford dried crude extract at the 3$^{rd}$ step; and subjecting to at least one purification process selected from (i) reverse phase partition chromatography, (ii) flash column chromatography, (iii) RP C18 column chromatography, (iv) Silica gel column chromatography, (v) ion exchange chromatography or (iv) size exclusion chromatography repeatedly to afford novel compound (KS 534) of the present invention.

The term "pharmaceutically acceptable carriers or excipients" defined herein comprises "pharmaceutical additives, the inactive ingredients used to make up a medication. They include dyes, flavors, binders, emollients, fillers, lubricants, preservatives, and many more classifications. Common excipients include cornstarch, lactose, talc, magnesium stearate, sucrose, gelatin, calcium stearate, silicon dioxide, shellac and glaze, which has been well-known in the art (*See,* Home-page of Food and Drug Administration: www.fda.gov or drug information online: www.drugs.com) or previous literature (for example, Rowe, Raymond C et al., Handbook of Pharmaceutical Excipients, Pharmaceutical Press, 7th Edition, 2012)

The term "prevent" disclosed herein comprises any act to inhibit or postpone the occurrence of certain disease or disorder disclosed herein by way of administrating the inventive composition; and the term "treat" disclosed herein comprises any act to alleviate or favorably changing the symptom associated with certain disease or disorder disclosed herein by way of administrating the inventive composition.

The present inventors have found that the novel compound (KS 534) may be prepared from *Pseudolysimachion* rotundum var subintegrum showed potent anti-inflammatory, anti-allergy, anti-asthma and anti-COPD activity through various in vitro tests, for example, (1) cyto-toxicity test using by HT1080, H292 and EL4 cell line, (2) an inhibition test on the expression of MUC5AC (oligomeric muscus/ gel-forming) induced by TNF-alpha, (3) NF-kappa B luciferase reporter assay, (4) inhibition test on the activity of NF-kappa B transcription factor; (5) inhibition on mRNA expression of target genes such as MMP-9, MUC5AC and IL-4 by way of inhibiting the NF-kappa B activity; (6) dose-dependent inhibiting effect on MUC5AC reproduction through MUC5AC protein reproduction assay; as well as in vivo tests, for example, (7) reducing effect on the number of inflammatory cells such as eosinophils using by OVA-sensitized/challenged mouse model, (8) inhibition test on the release of IgE, inflammatory cytokines such as IL-4, IL-5, IL-13 etc in BALF fluid and infiltration of inflamed cells, as well as the suppression of airway hyperresponsiveness and goblet cell hyperplasia, (9) an inhibition test using by COPD animal model (C57B/6N mouse) on the proliferation of inflammatory cells in BALF fluid, the inhibition on the reproduction of ROS (Reactive oxygen species), and activity of neutrophil elastase, the decreasing effect on the level of IL-6, TNF-alpha and the infiltrated inflammatory cells etc.

Accordingly, in accordance with the other aspect of the present invention, present invention provide a pharmaceutical composition or a health functional food comprising the novel compound (KS534) isolated from the extract of *Pseudolysimachion* rotundum var *subintegrum* as an active ingredient in an effective amount to treat and prevent allergic disease, inflammatory disease, asthma or COPD disease.

Present invention provide a pharmaceutical composition or a health functional food comprising the novel compound (KS534) isolated from the extract of *Pseudolysimachion* rotundum var subintegrum and the pharmaceutically acceptable carriers or excipients, for the treatment or prevention of allergic disease, inflammatory disease, asthma or chronic obstructive pulmonary disease (COPD).

In accordance with another aspect of the present invention, there is also provided a use of the novel compound (KS534) isolated from the extract of *Pseudolysimachion* rotundum var subintegrum for manufacture of medicines employed for treating or preventing allergic disease, inflammatory disease, asthma or chronic obstructive pulmonary disease (COPD).

In accordance with another aspect of the present invention, there is also provided a use of the novel compound (KS534) isolated from the extract of *Pseudolysimachion* rotundum var subintegrum and the pharmaceutically acceptable carriers or excipients for manufacture of medicines employed for treating or preventing allergic disease, inflammatory disease, asthma or chronic obstructive pulmonary disease (COPD).

In accordance with another aspect of the present invention, there is also provided a method of treating or preventing allergic disease, inflammatory disease, asthma or chronic obstructive pulmonary disease (COPD) in mammal, wherein the method comprises administering a therapeutically effective amount of the novel compound (KS534) isolated from the extract of *Pseudolysimachion* rotundum var subintegrum into the mammal suffering from allergic disease, inflammatory disease, asthma or chronic obstructive pulmonary disease (COPD).

In accordance with another aspect of the present invention, there is also provided a method of treating or preventing allergic disease, inflammatory disease, asthma or chronic obstructive pulmonary disease (COPD) in mammals, wherein the method comprises administering a composition comprising therapeutically effective amount of the novel compound (KS534) isolated from the extract of *Pseudolysimachion* rotundum var subintegrum and the pharmaceutically acceptable carriers or excipients, into the mammal suffering from allergic disease, inflammatory disease, asthma or chronic obstructive pulmonary disease (COPD).

The inventive composition for treating and preventing allergic disease, inflammatory disease, asthma or chronic obstructive pulmonary disease (COPD) may comprises above compounds as 0.1 ~ 99%, preferably, 0.1 ~ 50% by weight based on the total weight of the composition.

The composition according to the present invention can be provided as a pharmaceutical composition containing pharmaceutically acceptable carriers, adjuvants or diluents, e.g., lactose, dextrose, sucrose, sorbitol, mannitol, xylitol, erythritol, maltitol, starches, acacia rubber, alginate, gelatin, calcium phosphate, calcium silicate, cellulose, methyl cellulose, polyvinyl pyrrolidone, water, methylhydroxy benzoate, propylhydroxy benzoate, talc, magnesium stearate and mineral oil. The formulations may additionally include fillers, anti-agglutinating agents, lubricating agents, wetting agents, flavoring agents, emulsifiers, preservatives and the like. The compositions of the invention may be formulated so as to provide quick, sustained or delayed release of the active ingredient after their administration to a patient by employing any of the procedures well known in the art.

For example, the compositions of the present invention can be dissolved in oils, propylene glycol or other solvents that are commonly used to produce an injection. Suitable examples of the carriers include physiological saline, polyethylene glycol, ethanol, vegetable oils, isopropyl myristate, etc., but are not limited to them. For topical administration, the extract of the present invention can be formulated in the form of ointments and creams.

Pharmaceutical formulations containing present composition may be prepared in any form, such as oral dosage form (powder, tablet, capsule, soft capsule, aqueous medicine, syrup, elixirs pill, powder, sachet, granule), or topical preparation (cream, ointment, lotion, gel, balm, patch, paste, spray solution, aerosol and the like), or injectable preparation (solution, suspension, emulsion).

The composition of the present invention in pharmaceutical dosage forms may be used in the form of their pharmaceutically acceptable salts, and also may be used alone or in appropriate association, as well as in combination with other pharmaceutically active compounds.

The desirable dose of the inventive compound varies depending on the condition and the weight of the subject, severity, drug form, route and period of administration, and may be chosen by those skilled in the art. However, in order to obtain desirable effects, it is generally recommended to administer at the amount ranging from 0.0001 to 1000mg/kg, preferably, 0.001 to 100mg/kg by weight/day of the inventive extract of the present invention. The dose may be administered in single or divided into several times per day.

The pharmaceutical composition of present invention can be administered to a subject animal such as mammals (rat, mouse, domestic animals or human) *via* various routes. All modes of administration are contemplated, for example, administration can be made orally, rectally or by intravenous, intramuscular, subcutaneous, intracutaneous, intrathecal, epidural or intracerebroventricular injection.

The present invention also provides a pharmaceutical composition and a health food comprising the novel compound (KS534) isolated from the extract of *Pseudolysimachion* rotundum var subintegrum as an active ingredient in an effective amount to treat and prevent allergic disease, inflammatory disease, asthma or COPD disease.

The present invention also provides a use of the novel compound (KS534) isolated from the extract of *Pseudolysimachion* rotundum var subintegrum for manufacture of medicines employed for treating or preventing inflammatory disease, allergic disease, asthma or COPD.

The present invention also provides a method of treating or preventing inflammatory disease, allergic disease, asthma or COPD in a mammal or human comprising administering to said mammal or human an effective amount of the novel compound (KS534) isolated from the extract of *Pseudolysimachion* rotundum var subintegrum, together with a pharmaceutically acceptable carrier thereof.

The inventive extract of the present invention also can be used as a main component or additive and aiding agent in the preparation of various functional health food and health care food.

Accordingly, it is the other object of the present invention to provide a health functional food comprising a therapeutically effective amount of the novel compound (KS534) isolated from the extract of *Pseudolysimachion* rotundum var subintegrum containing active ingredients for the prevention or alleviation of allergic disease, inflammatory disease, asthma or chronic obstructive pulmonary disease (COPD).

The term "a functional health food" defined herein" the functional food having enhanced functionality such as physical functionality or physiological functionality by adding the extract of the present invention to conventional food to prevent or improve the purposed diseases in human or mammal.

It is the other object of the present invention to provide a health care food comprising a therapeutically effective amount of the novel compound (KS534) isolated from the extract of *Pseudolysimachion* rotundum var *subintegrum,* together with a sitologically acceptable additive for the prevention or alleviation of allergic disease, inflammatory disease, asthma or chronic obstructive pulmonary disease (COPD).

The term "a health care food" defined herein "the food containing the extract or compound(s) of the present invention showing no specific intended effect but general intended effect in a small amount of quantity as a form of additive or in a whole amount of quantity as a form of powder, granule, capsule, pill, tablet etc.

The term "a sitologically acceptable additive" defined herein comprises "any substance the intended use which results or may reasonably be expected to result-directly or indirectly-in its becoming a component or otherwise affecting the characteristics of any food", and can be classified into three groups according to its origin, i.e., (1) chemically synthetic additive such as ketones, glycin, potassium citrate, nicotinic acid, etc; (2) natural additive such as persimmon dye, licorice extract, crystalline cellulose, gua dum etc; (3) the mixed additive therewith such as sodium L-glutamate, presevatives, tar dye etc, or various categories according to its function in the food, for example, thickening agent, maturing agent, bleaching agent, sequestrant, humectant, anti-caking agent, clarifying agents, curing agent, emulsifier, stabilizer, thickener, bases and acid, foaming agents, nutrients, coloring agent, flavoring agent, sweetner, preservative agent, antioxidant, etc, which has been well-known in the art or previous literature (*See,* "Codex General Standard for Food Additives" (GSFA, Codex STAN 192-1995) in Home-page of GSFA Online: www.codexalimentarius.net/gsfaonline/index.html).

If a substance is added to a food for a specific purpose in that food, it is referred to as a direct additive and indirect food additives are those that become part of the food in trace amounts due to its packaging, storage or other handling.

The term "health care foods or health functional foods" disclosed herein can be contained in food, health beverage, dietary supplement etc, and may be formulated into a form of pharmaceutically dosing form such as a powder, granule, tablet, suspension, emulsion, syrup, chewing tablet, capsule, beverage etc; or the food form, for example, bread, rice cake, dry fruit, candy, chocolate, chewing gum, ice cream, milk such as low-fat milk, lactose-hydrolyzed milk, goat-milk, processed milk, milk product such as fermented milk, butter, concentrated milk, milk cream, butter oil, natural cheese, processed cheese, dry milk, milk serum etc, processed meat product such as hamburger, ham, sausage, bacon etc, processed egg product, fish meat product such as fish cake etc, noodle products such as instant noodles, dried noodles, wet noodles, fried noddles, non-fried noodles, gelatinized dry noodles, cooked noodles, frozen noodles, Pasta etc, tea product such as tea bag, leached tea etc, health drinks such as fruit drinks, vegetable drinks, carbonated soft drinks, soymilk drinks, lactic beverage mixed beverage, etc, seasoning food such as soy sauce, soybean paste, red pepper paste, chunjang (a kind of fermented soybean product colored by caramel), cheonggukjang (natural fermented soybean by B. subtillis), mixed paste, vinegar, sauce, ketchup, curry, dressing etc, margarine, shortening, pizza etc, but not intended herein to limit thereto, for preventing or improving of purposed disease.

Also, above described extract or compound can be added to food or beverage for prevention and improvement of purposed disorder. The amount of above described extract or a compound(s) in food or beverage as a functional health food or health care food may generally range from about 0.01 to 100 w/w % of total weight of food for functional health food composition. In particular, although the preferable amount of the extract of the present invention in the functional health food, health care food or special nutrient food may be varied in accordance to the intended purpose of each food, it is preferably used in general to use as an additive in the amount of the extract or a compound(s) of the present invention ranging from about 0.01 to 5% in food such as noodles and the like, from 40 to 100% in health care food on the ratio of 100% of the food composition.

Providing that the health beverage composition of present invention contains above described extract or a compound(s) as an essential component in the indicated ratio, there is no particular limitation on the other liquid component, wherein the other component can be various deodorant or natural carbohydrate etc such as conventional beverage. Examples of aforementioned natural carbohydrate are monosaccharide such as glucose, fructose etc; disaccharide such as maltose, sucrose etc; conventional sugar such as dextrin, cyclodextrin; and sugar alcohol such as xylitol, and erythritol etc. As the other deodorant than aforementioned ones, natural deodorant such as taumatin, stevia extract such as levaudiosideA, glycyrrhizin *et al.,* and synthetic deodorant such as saccharin, aspartam *et al.,* may be useful favorably. The amount of above described natural carbohydrate is generally ranges from about 1 to 20 g, preferably 5 to 12 g in the ratio of 100 mℓ of present beverage composition.

The other components than aforementioned composition are various nutrients, a vitamin, a mineral or an electrolyte, synthetic flavoring agent, a coloring agent and improving agent in case of cheese, chocolate *et al.,* pectic acid and the salt thereof, alginic acid and the salt thereof, organic acid, protective colloidal adhesive, pH controlling agent, stabilizer, a preservative, glycerin, alcohol, carbonizing agent used in carbonate beverage *et al.* The other component than aforementioned ones may be fruit juice for preparing natural fruit juice, fruit juice beverage and vegetable beverage, wherein the component can be used independently or in combination. The ratio of the components is not so important but is generally range from about 0 to 20 w/w % per 100 w/w % present composition. Examples of addable food comprising aforementioned extract or compound therein are various food, beverage, gum, vitamin complex, health improving food and the like.

Inventive extract or a compound(s) of the present invention has no toxicity and adverse effect therefore; they can be used with safe.

It will be apparent to those skilled in the art that various modifications and variations can be made in the compositions, use and preparations of the present invention without departing from the scope of the invention.

The present invention is more specifically explained by the following examples. However, it should be understood that the present invention is not limited to these examples in any manner.

**Advantageous Effects of Invention**

As described in the present invention, inventive the novel compound (KS534) isolated from the extract of *Pseudolysimachion* rotundum var subintegrum*subintegrum* showed potent anti-inflammatory, anti-allergy, anti-asthma and anti-COPD activity through various in vitro tests, for example, (1) cyto-toxicity test using by HT1080, H292 and EL4 cell line, (2) an inhibition test on the expression of MUC5AC (oligomeric muscus/gel-forming) induced by TNF-alpha, (3) NF-kappa B luciferase reporter assay, (4) inhibition test on the activity of NF-kappa B transcription factor; (5) inhibition on mRNA expression of target genes such as MMP-9, MUC5AC and IL-4 by way of inhibiting the NF-kappa B activity; (6) dose-dependent inhibiting effect on MUC5AC reproduction through MUC5AC protein reproduction assay; as well as in vivo tests, for example, (7) reducing effect on the number of inflammatory cells such as eosinophils using by OVA-sensitized/challenged mouse model, (8) inhibition test on the release of IgE, inflammatory cytokines such as IL-4, IL-5, IL-13 etc in BALF fluid and invasion of inflamed cells, as well as the suppression of airway hyperresponsiveness and golblet cell hyperplasia, (9) an inhibition test using by COPD animal model (C57B/6N mouse) on the proliferation of inflammatory cells in BALF fluid, the inhibition on the reproduction of ROS (Reactive oxygen species), and activity of neutrophil elastase, the reducing effect on the level of IL-6, TNF-alpha and the infiltrated inflammatory cells etc.

**Brief Description of Drawings**

The above and other objects, features and other advantages of the present invention will more clearly understood from the following detailed description taken in conjunction with the accompanying drawings, in which;

Fig. 1 shows $^1H$-$^1H$ COSY and HMBC correlation of KS534 compound;
Fig. 2 shows the effect of test sample on the mRNA expression of MUC5AC using by qRT-PCR;
Fig. 3 shows the inhibition effect of test sample on the activity of TNF-alpha induced NF-kappa B using by luciferase reporter assay (Statistical analysis was performed by Student' s-t-test and *P* value (**, <0.01) was regarded as being statistical significant);
Fig. 4 presents the inhibition effect of test sample on the expression level of MUC5AC protein using by modified-ELISA method (Statistical analysis was performed by Student' s-t-test and *P* value (**, <0.01 and ***, <0.001) was regarded as being statistical significant);
Fig. 5 presents the effect of the inventive compound on the number of inflammatory cells in BALF;
Fig. 6 presents the effect of the inventive compound on the number of inflammatory cytokines in BALF;
Fig. 7 presents the effect of the inventive compound on the number of total IgE and level of OVA-specific IgE in serum;
Fig. 8 presents the effect of the inventive compound on the inflammatory response in lung tissue cell using by the histological examination;
Fig. 9 presents the effect of the inventive compound on the mucosal hypersecretion in lung tissue cell using the histological examination;
Fig. 10 presents the effect of the inventive compound on the number of inflammatory cells in BALF;
Fig. 11 presents the effect of the inventive compound on the reproduction of ROS in BALF;
Fig. 12 presents the effect of the inventive compound on the level of BALF contents and elastase activity in BALF;
Fig. 13 represents the effect of the inventive compound on the level of IL-6 and TNF-alpha in BALF;
Fig. 14 depicts the effect of the inventive compound on the inflammatory response in lung tissue cell using the histological examination;

**Best Mode for Carrying out the Invention**

It will be apparent to those skilled in the art that various modifications and variations can be made in the compositions, use and preparations of the present invention without departing from the spirit or scope of the invention.

The present invention is more specifically explained by the following examples. However, it should be understood that the present invention is not limited to these examples in any manner.

**EXAMPLES**

The following Reference Example, Examples and Experimental Examples are intended to further illustrate the

present invention without limiting its scope.

**Reference Example 1. Analysis Apparatus**

Melting point was determined with no correction by melting point determination apparatus (Koflermicrohostage); optical rotation by Jasco P-1020 polarimeter; UV data by UV-VIS 2450 spectrometer; FT-IR spectra by Jasco FT/IR-4200; NMR spectra by Varian UNITY 400 MHz FT-NMR spectrometer using by TMS as an internal standard; HRESIMS by Waters Q-TOF Premier spectrometer; and HPLC analysis by Gilson HPLC using by UV/VIS-155 detector and pump 305 in the experiment.

**Example 1. Preparation of novel compound (KS 534) from** *Pseudolysimachion rotundum* var *subintegrum*

1-1. Preparation of crude extract

2.0 kg of dried *Pseudolysimachion* rotundum var subintegrum (cultivated at 244, Soi-myeon Eumseong-gun Chungcheongbuk-do in Korea according to GAP, KRIBB 0020697, plant extract bank of KRIBB, Taejeon, KOREA) cut into small pieces and mixed with 10L of 40% ethanol. The mixture was stirred at room temperature for 24 hours and extracted with reflux extraction at 78°C for 12 hours to collect the filtrate, three times. The extract was filtered with filter paper to remove the debris. The collected filtrate was concentrated by rotary evaporator (EYELA, N-2100, Japan) at 55~65°C under reduced pressure and dried with freezing dryer to obtain 198.7g of dried crude extract

1-2. Preparation of purified extract

20 g of dried crude extract was dissolved in mixture solvent (25% MeOH-water) and subjected to further purification using by preparative reverse phase chromatography (Zeoprep C18, 75 μm, 200 x 250 mm, Zeochem, Louisville, U. S. A). The eluting fractions (f1- f4) were collected and concentrated under vaccuo. 5.0 g of the fraction f2 was loaded to medium pressure liquid chromatography (MPLC) using by column: RP C-18 (Zeoprep C18, 20 x 250 mm, 10 μm, Zeochem, Louisville, U. S. A.) and eluting solvent (MeOH-water solution = 2:8, 3:7, 4:6, 10:0) to afford 5 sub-fractions, f2a, f2b, f2c, f2d and f2e.

1-3. Preparation of novel compound KS534

0.8 g of sub-fraction (f2c) was subjected to semi-preparative HPLC (Synergy Polar-RP 4 μm, 21.2 x 250 mm, Phenomenex, Torrance, CA, U.S.A., 22% MeCN in $H_2O$) to obtain bright brown powdered novel iridoid compound (3R,5S,5aS,6R,7S,8R,8aS)-8-chloro-8a-hydroxy-5-(2-hydroxy acetoxy) hexahydro-1H-3,6-methanocyclopenta[e][1,3] dioxepin-7-yl 3,4-dihydroxybenzoate (KS534; $C_{22}H_{26}ClO_{13}$) showing physic-chemical property.

$[\alpha]^{20}_D$ -30.0° (*c* 0.2, MeOH).

HRESIMS (observed *m/z* 533.1035 $[M-H]^-$): quasimolecular 3:1 ion cluster

IR spectrum: 3412 $cm^{-1}$ (hydroxyl group); 1692 $cm^{-1}$. (unsaturated ester carbonyl)

$^1H$ and $^{13}C$ NMR spectra: Table 1

$^1H$-$^1H$ COSY spectrum (Fig. 1)

NOESY spectrum (Fig. 1)

[Table 1]

| $^1H$ (400 MHz) and $^{13}C$(100MHz) NMR spectroscopic data for KS-534 in DMSO-*d6* | | |
|---|---|---|
| Position | KS-534 | |
| | $\delta_C$ | $\delta_H$(*J*inHz) |
| Agluc | | |
| 1 | 90.6 | 5.55, d (1.6) |

(continued)

| $^1H$ (400 MHz) and $^{13}C$(100MHz) NMR spectroscopic data for KS-534 in DMSO-*d6* | | |
|---|---|---|
| Position | KS-534 | |
| | $\delta_C$ | $\delta_H$(*J*inHz) |
| 3 | 93.5 | 5.30, d (2.4) |
| 4 | 32.9 | 2.38, dd (13.4, 8.2) |
| | | 1.94, dd (13.4, 2.4) |
| 5 | 33.0 | 2.26, ddd (9.6, 8.8, 2.2) |
| 6 | 85.7 | 4.96, dd (8.8, 2.2) |
| 7 | 69.2 | 4.54, d (8.8) |
| 8 | 78.3 | |
| 9 | 46.4 | 2.55, br d (10.0) |
| 10 | 60.8 | 3.56, d (12.4) |
| | | 3.86, d (12.4) |
| 11 | | |
| Glc | | |
| 1' | 96.8 | 4.50, d (7.6) |
| 2' | 73.0 | 2.91, d (8.4) |
| 3' | 76.5 | 3.13, m |
| 4' | 70.1 | 3.04, t (9.2) |
| 5' | 77.1 | 3.13, m |
| 6' | 60.9 | 3.69, d (8.8) |
| | | 3.43, dd (12.0, 5.6) |
| Aroyl | | |
| 1" | 119.8 | |
| 2" | 116.4 | 7.39, d (2.1) |
| 3" | 145.1 | |
| 4" | 150.9 | 6.83, d (8.0) |
| 5" | 115.4 | 7.37, dd (8.0, 2.1) |
| 6" | 122.1 | |
| 7" | 165.6 | |

**Experimental Example 1. Preliminary determination of the cytotoxicity.**

In order to determine the cytotoxicity of inventive compound, following preliminary test was performed by the method disclosed in the literature (Shiyama et al., Talanta, 1997, 44, 1299-1305.; Tominaga et al., Anal. Commun., 1999, 36, 47-50).

1-1. Preparation of cell line and cell culture

HT1080 (CRL-12012), H292 (CRL-1848) and murine EL4 cells (TIB-39) were purchased from company (American Type Culture Collection).

HT1080 (CRL-12012) and murine EL4 cells were cultured in DMEM medium (SB30243.01, Dulbecco's modified Eagle's medium; DMEM, HyClone) supplemented with 10% FBS (Fetal bovine Sreum; Gibco) and antibiotic (100 units/ml

penicillin + 100 units/ml streptomycin). H292 cell was cultured in RPMI medium (SH30027.01, RPMI 1640, Gibco) supplemented with 10% FBS and antibiotic. The cells were cultures under the condition of humidified 5% $CO_2$ atmosphere at 37°C. PMA (P8139, Phorbol 12-myristate 13-acetate) was purchased from the company (Sigma Chemical Co., St. Louis, MO) and TNF-α is from the company (300-01A, PeproTech, NJ USA Inc.) to use in the experiment.

1-2. Evaluation of cytotoxicity in H292 cell

H292 cell line, a human hung mucosal cancer cell line, was suspended in 10% FBS-supplemented RPMI medium in a concentration of 5 x $10^4$cells/ml, and 100μL of the suspension was inoculated into 96 well plates to adhere cell to the plate for 12 hours. Various concentrations of test sample (KS 534 compound) were treated therewith and cultured for 24 hours. 10 μL of CCK-8 solution was mixed with 90 μL of the medium and 100 μL of solution was added to each well. After reacting from 30 mins to 4 hours, the absorbance of the solution was determined at 570 nm. The cell viability was calculated according to following Math formulae 1 based on negative control group treated with 0.2% DMSO and the result was shown in Table 2.

[Math formulae 1]

Cell viability (%) = OD 570nm (test group)/ OD 570nm (negative control group) x 100

At the result, as shown in Table 2, it has been confirmed that KS 534 compound did not show cytotoxicity in H292 cell at less than 20 μM.

[Table 2]

| Test sample | concentration(μM) | H292 cell viability(%, mean±variation) |
|---|---|---|
| Negative control group | 0 | 100.00 ± 2.50 |
| KS-534 | 2.5 | 101.88 ± 3.71 |
| | 5 | 102.46 ± 3.96 |
| | 10 | 99.89 ± 4.79 |
| | 20 | 97.87 ± 2.92 |

**Experimental Example 2. Inhibition on the expression of** MUC5AC.

In order to determine the inhibitory effect on the expression of MUC5AC induced by TNF-alpha in H292 cell line, following test was performed:

2-1. determination of the level of mRNA expression

The isolation of total RNA, cDNA synthesis and quantitative real-time PCR were performed by the method disclosed in the literature (Kim et al., J. Cell. Biochem.2008, 104, 1906-1917).

In summary, total RNA was isolated using by TRIzol reagent (155596-026, Life Technologies) and the first strand cDNA was synthesized using by enzyme (Omniscript Reverse Transcriptase, 205113, Qiagen, CA). The quantitative real-time PCR amplification was performed using by apparatus 1 (S1000 Thermal cycler, Bio-rad, USA) and apparatus 2 (2 x Greenstar master mix, Bioneer, Korea) according to the manufacture's manual.

2-2. Effect on MUC5AC expression

In order to determine the inhibitory effect on the expression of MUC5AC induced by TNF-alpha in H292 cell line, following test was performed.

The inhibitory effect of KS534 compound on MUC5AC mRNA expression was determined to verify the treating or preventing activity of inflammatory disease such as COPD.

Specifically, the reproduced amount of TNF-alpha induced MUC5AC was determined by Real-time polymerase chain reaction (real-time PCR), quantitative real time polymerase chain reaction (qPCR) and MUC5AC immunoassay.

H292 cells were inoculated into 48 well plates in a concentration of 2 x $10^4$ cells/well to adhere for 24 hours and the culture medium was replaced with new 0.1% FBS containing medium to culture for 24 hours. 10μM KS-534 was treated with therewith for 2 hours and 20ng/ml of TNF-alpha was treated therewith to culture for 12 hours.

In order to extract total RNA, RNA was extracted using by Trizol B (Invitrogen) and cDNA was synthesized using by Omniscript RT kit (Qiagen, GmbH, Hilden, Germany) after the quantification. The synthesized cDNA was mixed with the template, MUC5AC and GAPDH primers as shown in Table 3, respectively, and performed to PCR using by PCR mix (PCR Master Mix, Bioneer, Korea) as follows: 5 mins at 94°C, 1cycle for pre-denaturation; 30 sec at 95°C, 45 sec at 60°C and 45 sec at 72°C, 30 cycles for denaturation; 10 mins at 72°C, 1cycle for final extension.

[Table 3]

| Gene (species) | primer | |
|---|---|---|
| MUC5AC (Human) | sense | 5'-TGA TCA TCC AGC AGC AGG GCT-3' |
| | antisense | 5'-CCG AGC TCA GAG GAC ATA TGG G-3' |
| GAPDH (Human) | sense | 5'-CGG AGT CAA CGG ATTT GGT CGT AT - 3' |
| | antisense | 5'-AGC CTT CTC CAT GGT GGT GAA GAC-3' |

At the result, it has been confirmed that the amount of MUC5AC was increased in the control group treated with TNF-alpha and the amount of mRNA expression of MUC5AC in the test group treated with 10 μM was decreased by 58.47% (inhibitory ratio of KS-534 on the MUC5AC expression: 41.52%) as can be seen in Table 4 and Fig. 2.

Accordingly, it has been verified that KS-534 compound inhibit the gene expression of MUC5AC and it is useful in treating COPD.

[Table 4]

| The summary for inhibitory activity of Muc5AC mRNA expression by KS-534 | | | | |
|---|---|---|---|---|
| sample | concentration(μM) | TNF-α (20 ng/mL) | Expressed level of MUC5AC mRNA (Relative % comparing with that in TNF-alpha treatment group) | Inhibitory ratio (%) |
| Negative control | 0 | - | 7.02 ± 2.82 | 92.82 |
| TNF-alpha treatment | 0 | + | 100.00 ± 25.35 | 0.00 |
| KS-534 | 10 | + | 58.47 ± 18.50 | 41.52 |

**Experimental Example 3. Inhibition on the activity of NF-kappa B.**

In order to determine the inhibitory effect on the activity of NF-kappa B, following test was performed:

3-1. NF-kappa B luciferase reporter assay

NF-kappa B luciferase reporter assay was performed by following procedure disclosed in the literature (Pulin Che et al., Assay Drug Dev. Technol., 2012, 10, 61-68).

In order to establish the stable cells for NF-kappa B luciferase reporter assay, NF-kappa B response element was cloned into vector (pGL4.32vector, E849A, Promega) according to the manufacture's protocol and infected to the cell (H292 cells, CRL-1848, ATCC) using by Lipofectamine 2000 (11668-019, Invitrogen). The infected cell was selected using by hygromycin (400 μg/mL) for 2 weeks and the colonies were individually cloned and proliferated. The cell was placed on the 96-well plates in a concentration of 5×$10^3$cell/well for 24 hours. The test sample was pre-treated in a predetermined concentration and 20 ng/ml of TNF-alpha was treated therewith for 12 hours. The activity of luciferase enzyme was determined by E6110 kit (OneGlo Luciferase Assay kit, Promega) and DMSO was used as a vehicle control.

3-2. Inhibitory effect on the activation of NF-kappa B transcription factor

It has been reported that TNF-alpha activate the NF-kappa B transcription factor to induce MUC5AC expression

(Busse, P. J. et al., J. Allergy Clin. Immun., 2005, 116, 1256-1263.; Smirnova, M. G. et al., Cytokine. 2000, 12, 1732-1736).

Based on the inhibitory effect of KS-534 compound on MUC5AC expression, the inventors have studied to confirm whether the activity of NF-kappa B transcription factor presented in MUC5AC promoter is inhibited by KS-534 compound.

The stably expressed H292 cells, a normal human bronchial epithelium cell, with luciferase reporter vector pGL4.32 (luc2P/NF-KB-RE/Hygro, Promega Company) were established prior to following experiment. The cell was suspended in 10% containing DMEM medium (Hyclone Company) in a concentration of $5\times10^4$cells/mL and each 100 μL of cell was seeded on 96 well plates to adhere for 12 hours to the plates. The medium was replaced with new 0.1 % FBS containing medium to culture 12 hours and 10 μM KS-534 was treated for 2 hours. 20 ng/ml of TNF-alpha was treated therewith to culture for 12 hours. The activation degree of luciferase was determined by One-glow Luciferase Assay system (Promega Company). After removing all the culture media in well plate, the plate was washed with PBS solution twice and 60μL of passive lysis buffer (PLB) was added to each well to lyse the cell for 30 mins. 50μL of lysed solution of the cell was transferred to new 96 well plates and 50 μL of LAR II reagent containing luciferase substrate of fireflies was added thereto. The luminescence was determined by Microplate Luminometer LB 96V (EG&G Berthold company) for 0.5 sec and the result was shown in Table 5 and Fig. 3.

[Table 5]

| The summary for inhibition rate of NF-kappa B activity by KS-534 | | | | |
|---|---|---|---|---|
| sample | Conc. (μM) | TNF-alpha(20 ng/mL) | NF-kappa B activity(relative % comparing with TNF-alpha treatment group) | Inhibitory ratio(%) |
| Negative control | 0 | - | 34.05 ± 1.99 | - |
| TNF-alpha treatment | 0 | + | 100.00 ± 6.22 | 0.00 |
| KS-534 | 10 | + | 63.35 ± 6.18 | 36.65 |

At the result, it has been confirmed that the test group treated with 10 μM KS-534 showed a potent inhibitory effect by 63.35 % (inhibitory ratio of KS-534; 36.65 %) based on NF-kappa B activity induced by TNF-alpha (100 %).

Accordingly, it has been verified that KS-534 compound significantly inhibited the activity of NF-kappa B transcription factor.

**Experimental Example 4. Inhibition on the reproduction of MUC5AC protein.**

In order to determine the inhibitory effect on the reproduction of MUC5AC protein, following test was performed by the method disclosed in the literature (Sikder, MA. et al., Phytother. Res., 2014, 28, 62-68)

4-1. Inhibitory effect on reproduction of MUC5AC protein

In order to determine the treating activity of inflammatory disease such as COPD, the inhibitory effect on the release of MUC5AC was determined as follows:

For the immunoassay on reproduced MUC5AC, 50 μL of collected supernatant in Experimental Example 3 was distributed to 96 well plate and dried in a thermostat at 50 °C.

After washing with 1 % BSA added PBS solution, MUC5AC antibody (ab3649, abcam Co.) was added to react together at room temperature for 1 hour and then secondary antibody was added thereto to react together for 1 hour. After re-washing, 3, 3', 5, 5'-tetramethylbenzidine peroxide solution (54827-17-7, Sigma-aldrich) was added thereto to react together for 20 mins. After stopping the reaction by adding sulfuric acid solution, the absorbance was determined by VERSAmax microplate reader, (SMP500-14915, Molecular Devices, USA) at 450 nm (Sikder, M. A. et al., Phytother. Res., 2014, 28, 62-68; Takeyama, K. et al.Proc. Natl. Acad. Sci. U.S.A., 1999, 96, 3081-3086). The result was shown in Table 6 and Fig. 4.

At the result, it has been confirmed that the secreted amount of MUC5AC in the group treated with TNF-alpha was significantly increased and the test group treated with 2.5, 5 and 10μM KS-534 showed potent inhibitory effect by 93.38, 78.27 and 64.77 % (inhibitory ratio of KS-534; 6.62, 21.73 and 35.23 %) on the reproduction of MUC5AC based on the secreted amount of MUC5AC in the group treated with TNF-alpha (100 %).

[Table 6]

| The summary for inhibitory activity of Muc5AC secretion by KS-534 | | | | |
|---|---|---|---|---|
| sample | Conc. (μM) | TNF-alpha (20 ng/mL) | MUC5AC secretion(relative % comparing with TNF-alpha treatment group) | Inhibitory ratio (%) |
| Negative control group | 0 | - | 25.98 ± 0.88 | - |
| TNF-alpha treatment | 0 | + | 100.00 ± 1.22 | 0.00 |
| KS-534 | 2.5 | + | 93.38 ± 0.90 | 6.62 |
| | 5 | + | 78.27 ± 2.16 | 21.73 |
| | 10 | + | 64.77 ± 1.56 | 35.23 |

**Experimental Example 5. Anti-asthma activity in asthma-induced animal model.**

In order to determine the anti-asthma activity of test sample in asthma-induced animal model, following test was performed by the method disclosed in the literature (Shin, I. S. et al.,Food Chem. Toxicol.,2013, 62, 506-513)

5-1. Animal sensitization and airway challenge

Specific pathogen-free female BALB/c mice (about 20g), aged 6 weeks, which were routinely screened serologically for relevant respiratory pathogens, were purchased from Samtako Co. (Seoul, Korea) and acclimated with the experimental environment under the condition controlling the temperature of 22 ± 2°C, and relative humidity of 55 ± 15°C, at light/dark cycle for 1 week prior to experiment.

Briefly, mice were sensitized by intraperitoneal injection of 20 μg OVA(Ovalbumin; A5503, Sigma, St. Louis, MO), which was emulsified in 2 mg aluminum hydroxide (A8222, Sigma-Aldrich, MO. USA) in 200 μl of PBS buffer (pH 7.4), biweekly. The mice were challenged through the airways with OVA (1 % in PBS) for 30 min using an ultrasonic nebulizer (NE-U12; Omron Corp., Tokyo, Japan) from the $21^{st}$ day to $23^{rd}$ day after the initial sensitization. 24 hrs after the antigen treatment, the airway hyperresponsiveness was determined and the mice were sacrificed 48 hrs after the last challenge. The mice were sacrificed with an overdose of pentobarbital (50 mg/kg, Entobal®, Hanil, Korea) 48h after the last challenge, and a tracheotomy was performed. After 1.4ml of physiological saline solution (PBS) was instilled into the lungs, bronchoalveolar lavage fluid (BALF) was obtained by aspiration three times via tracheal cannulation.

The groups were divided into several groups, i.e., (a) normal control group(NC): the groups treated or not-treated with OVA; (b) asthma-induced group(OVA): the groups treated with OVA to induce asthma; and (c) comparative group: the groups treated with positive control group (M30, montelukast; 30 mg/kg, PO, Sigma-Aldrich Co. Ltd., SML-0101+OVA) and (d) test sample group: the groups treated with KS534 compound (30 mg/kg, PO, Sigma-Aldrich Co. Ltd., SML-0101+OVA).

The test group consists of 7 mice for each group and various concentrations of the test sample were orally administrated to the mice from $21^{th}$ to $23^{rd}$ day after $1^{st}$ OVA treatment.

All the result obtained from various experiments was recorded as mean± SD (standard deviation), and the comparison between each group was determined using by one-way ANOVA test using by SPSS 10.0 program. The statistic significance between each group was determined according to Dunnett's multiple comparison tests as a post-hoc test). The result was expressed as P values: < 0.05 (*) as statistically significant.

5-2. Isolation of BALF and determination of immunocyte

The supernatant of bronchoalveolar lavage fluid (BALF) recovered from each mice was isolated using by centrifuge and kept in refrigerator to determine the level of inflammatory cells.

BALF was loaded onto a slide and centrifuged to fix the cells onto the slide using a Cellspin machine (Cyto12.5+clip5, Hanil Science Industrial, Korea). The cells were stained by Diff-Quick® Stain reagents (Sysmex, Cat No.38721, Switzerland) according to the manufacturer's instructions and the number of inflammatory cells in each sample was counted by microscope (x400).

As shown in Fig. 5, the total number of eosinophil and inflammatory cells in the control group treated and inhaled with OVA as an asthma induced group (OVA) was significantly increased comparing with those in the non-treatment group with OVA as a normal control group (NC).

The total number of eosinophil and inflammatory cells in the test sample group orally administrated with various concentrations of test sample was significantly reduced.

5-3. Analysis on level of cytokines in BALF

In order to confirm the inhibition effect of test samples on the level of Th2 cytokines (IL-4, IL-5 and IL-13) in bronchoalveolar lavage fluid (BALF), the level of Th2 cytokines (IL-4, IL-5 and IL-13) in bronchoalveolar lavage fluid (BALF) was determined using by ELISA kit (VersaMax, Molecular Devices, USA) specifically reacting with each cytokine according to the manufacture's manual.

As shown in Fig. 6, the level of Th2 cytokines (IL-4, IL-5 and IL-13) in the control group treated with OVA as an asthma induced group (OVA) was significantly increased comparing with those in the non-treatment group with OVA as a normal control group (NC).

The increased level of Th2 cytokines (IL-4, IL-5 and IL-13) in the positive control group treated with Montelukast (MO, 30 mg/kg) as well as the test sample group orally administrated with various concentrations of test samples (KS534) were significantly reduced.

5-4. Effect on the level of IgE and OVA-specific IgE in blood serum

In order to confirm the inhibition effect of test samples prepared in Examples on the level of IgE and OVA-specific IgE in blood serum, following ELISA test was performed by the method disclosed in the literature (Kay, A.B., N. Engl. J. Med., 2001, 344, 30-37).

The blood serum isolated from caudal vena cava prepared in the above was recovered to determine the level of IgE and OVA-specific IgE in blood serum.

The blood serum was added to 96-well plates (ELISA plate, 2592, Costa, USA) and coated with 0.1M $NaHCO_3$ buffer solution (pH 8.3) containing 20μg/ml of OVA (Sigma, MO, USA) at 4 for 16 hours. After inhibiting nonspecific reaction using by PBS containing 1% bovine serum albumin(P2007, Biosesang, Korea), the serum for testing was diluted to 1:400 and reacted together for 2 hours at room temperature. After washing with PBS containing 0.05% tween 20, the serum was reacted with diluted (x300) anti-mouse IgE monoclonal antibody (MCA419, Serotec, Oxford, UK) for 2 hours and with diluted (x4000) HRP-conjugated goat anti-rat IgG polyclonal A (432402, Biolegend, USA) for 1 hours at room temperature. After washing, the solution was stained with 3,3', 5,5'-tetramethylbenzidine (432402, Biolegend Ins, USA) substrate and the reaction was stopped by 2N $H_2SO_4$ to determine the absorbance using by spectroscopy (Versamax, Molecular Devices, US) at 450nm.

As shown in Fig. 7, the level of IgE and OVA-specific IgE in blood serum in the control group treated with OVA as an asthma induced group (OVA) was significantly increased whereas those in the positive control group treated with Montelukast (MO) as well as the test sample group orally administrated with various concentrations of test samples (KS-534) were significantly reduced.

5-5. Lung histology

In order to confirm the anti-asthmatic effect of test samples prepared in Examples, following histopathological analysis on broncho-alveolar tissue was performed by the method disclosed in the literature (Kwak YG. et al.,J. Clin. Invest., 2003, 111, 1083-1092).

The delivered lung tissues of BALB/c mice which had not perform broncho-alveolar lavage was fixed for 24 h in 10 % neutral-buffered formalin. After being embedded in epoxy, then made into 4μm thickness sections, the tissue was stained with H&E solution (hematoxylin; Sigma MHS-16 and eosin, Sigma HT110-I-32) to observe the inflammation of lung tissue and with periodic acid Schiff (PAS, IMEB Inc., USA) to observe the mucosal secretion in lung tissue. The pathological change of lung tissue was determined by optical microscopy.

As shown in Fig. 8, many inflammatory cells including eosinophils were found in bronchiolar surroundings and hyperplasia of epithelial cells as well as hypertrophy of tracheal muscle were also found in the control group treated with OVA as an asthma induced group(OVA) whereas the invasion of the inflamed cells was significantly reduced in the positive control group treated with Montelukast (MO, 30 mg/kg) as well as the test sample group orally administrated with various concentrations of test samples (KS 534).

As shown in Fig. 9, the mucosal secretion from the goblet cell of bronchial epithelial cells in lung tissue was sharply increased in the control group treated with OVA as an asthma induced group (OVA) whereas the mucosal secretion from the goblet cell of bronchial epithelial cells in lung tissue was significantly reduced in the positive control group treated with Montelukast (MO, 30 mg/kg) as well as the test sample group orally administrated with various concentrations of test samples (KS 534).

In summary, the inventive KS534 compound could prevent or treat various diseases such as asthma caused

by OVA. The inventive KS534 compound effectively inhibited the filtration of inflammatory cells including eosinophilia, the reproduction of Th2 type IL-4, 5, 13 as well as reduced the level of IgE and the reproduction of OVA-specific IgE, which has been confirmed by histopathological analysis on bronchoalveolar tissue.

In conclusion, it has been confirmed that the inventive KS534 compound effectively alleviated the main syndrome of asthma such as eosinophilia, lung inflammation, mucus hypersecretion as well as inhibited the immune response and therefore, it can be useful as a potent natural treating agent of asthma since it has similar or equivalent efficacy with conventionally available chemical drug, montelukast.

**Experimental Example 6. animal model test**

In order to determine the anti-COPD effect of inventive compounds in COPD animal model, following test was performed by using COPD induced mice as disclosed in the literature (Bhavsar, T. et al.,J. Chronic Obstr. Pulm. Dis.,2008, 3, 477-481).

6-1. Experiment animal

Specific pathogen-free male C57BL/6N mouse (about 22-24 g), aged 6 weeks, which were routinely screened serologically for relevant respiratory pathogens, were purchased from Koatech Co. (www.koatech.co.kr, Seoul, Korea) and bred allowing to access freely to feed (antibiotic free, Samyang Oil & Feed Corp., Korea) and water in breeding room controlling the temperature of 22 ± 2°C, and humidity of 55 ± 15 % at the light-dark cycle for 12 hours and acclimated with the experimental environment for 1 week.

6-2. Drug and Administration

(1) test sample

2 kinds of test samples, i.e., KS534 (15 and 30 mg/kg), Daxas (main ingredient: Roflumilast, 10mg/kg) were dissolved in 0.5% sodium carboxymethyl cellulose (C9481, Sigma-Aldrich, USA) and used as test samples.

(2) administration

KS534, and Rolflumilast (ROF II-492, HETERO, India) were orally administrated to the mice, 1 hour before prior the intratracheal instillation (i.t.).

6-3. Preparation of COPD mouse model

(1) standard cigarette

3R4F Kentuchy Reference Cigarettes (University of California, USA) was used as a standard cigarette for generating a cigarette smoke. The cigarette containing 9.4 mg of tar, 11 mg of TPM (total particle matter) and 12 mg of carbon monooxide per piece, was used after harmonizing with the temperature of 22 ± 1 °C and humidity of 60 ± 2 % after opening for 48 ~ 72 hrs.

(2) Procedure

The exposure of cigarette smoke was performed by using a cigarette smoke generator (DBL Co. Ltd., www.dhbiolink.com, Korea). The mice were divided into five groups: normal (NC), COPD (cigarette smoke with LPS intranasal instillation), Rof (10 mg/kg of roflumilast, p.o. + cigarette smoke with LPS intranasal instillation), and KS534-15 and 30 (15 mg/kg and 30 mg/kg of KS534, p.o. + cigarette smoke with LPS instillation, respectively). Exposure of cigarette smoke (one puff/min, 35 mL puff volume over 2 s, every 60 s, 8 cigarettes per day) was conducted using a cigarette smoke generator. The mice received 1 h of cigarette smoke exposure in an exposure chamber (50 cm × 30 cm × 30 cm) for 3 days. Roflumilast and piscroside C were administered to mice by oral gavage 1 h before cigarette smoke exposure for 3 days. LPS was intranasally instilled (10 μg dissolved in 50 μL distilled water) under anesthesia 1 h after the final exposure to cigarette smoke. After the end of experiment, the blood, BALF, and pneumonocyte of each rat were isolated and collected to test.

6-4. BALF isolation and determination of the number of immunocytes

After finishing the experiment, rats were anaethetized with Zoletil50 (3VX9, Virbac, France, p.o) and the blood was delivered through caudal veins. In order to isolate BALF from lung, the bronchus of right lung was ligated with suture and then performed to tracheostomy. IV-use catheter (16 GA, 3S-Cath, Dukwoo, Korea) was put into the bronchus, and both of bronchus and catheter (16 GA, 3S-Cath, Dukwoo, Korea) were fixed with suture. The injector containing 1mL of DPBS 0.7 (Dubecco's phosphate-buffered saline, Invitrogen, USA) was connected thereto and DPBS was forced to circulate three times to isolate BALF. The right lung ligated with suture was isolated, fixed with 10 % neutral formalin solution, and the remaining lung tissue was reserved in refrigerator at -70 °C. The isolated BALF was centrifuged for 15 mins at 1500 rpm to prepare cell pellet and the supernatant was reserved in refrigerator at -70 ° C for cytokine analysis. The cell pellet was suspended in DPBS, and the cell was attached to a slide glass using by cytospin centrifuge (CS03270047, Hanil, Korea). Diff-Quik staining (ZS1003, Sysmex, Japan) was performed and the cell was observed by optical microscopy (DM1000, Leica, German) to count the number of immunocyte in each test sample.

As shown in Fig. 10, the characteristic increased level of neutrophils in BALF was observed in COPD induced group. The drug control group treated with Roflumilast showed reduced level of neutrophils by about 21.1 % comparing with COPD induced group. In a while, the groups treated with 15 mg/kg and 30 mg/kg of KS534 showed remarkably reduced level of neutrophils by 26.3 % and 25.7 %, respectively, comparing with COPD induced group.

6-5. Effect on ROS and neutrophils elastase activity in BALF

The level of ROS in BALF isolated from the mice was determined by DCF-DA (dihydrodo chloro fluoresce indiacetate, 35854, Sigma-Aldrich, USA) and the final concentration of DCF-DA was adjusted to 20 μM in 200 μL to incubate 30 mins. The Fluorescence was determined by apparatus (Flexstation3, Molecular Device, USA). The activity of neutrophils elastase activity in BALF was determined by using N-succinyl-(Ala)3-p-nitroanilide(S4760, Sigma-Aldrich) as a substrate and incubated for 90 mins. The resulting absorbance was determined at 405nm.

As shown in Fig. 11, the reproduction of ROS (Reactive oxygen species) in BALF was sharply increased in COPD induced group. However, the drug control group treated with Roflumilast showed reduced level of ROS by about 22.2 % comparing with COPD induced group and the groups treated with 15 mg/kg and 30 mg/kg of KS534 showed remarkably reduced level of ROS by 19.5 % and 27.5 % comparing with COPD induced group.

As shown in Fig. 12, in COPD induced group, the level of proteins was sharply increased in BALF. The drug control group treated with Roflumilast showed significant reduction in the level of proteins compared with COPD induced group. The groups treated with 15 mg/kg and 30 mg/kg of KS534 showed remarkably reduced level of proteins by 20.8 % and 28.5 % comparing with COPD induced group.

As shown in Fig. 12, in COPD induced group, the activity of neutrophils elastase enzyme was sharply increased in BALF. The drug control group treated with Roflumilast showed significant reduction in the activity of neutrophils elastase enzyme compared with COPD induced group. The groups treated with 15 mg/kg and 30 mg/kg of KS534 showed remarkably reduced activity of neutrophils elastase enzyme comparing with COPD induced group.

6-6. Cytokine analysis in BALF

The level of IL-6 (SM6000B, R&D System, USA) and TNF-alpha (555268, BD Bioscience, USA)) in BALF isolated from the mice was determined by enzyme-linked immuno-sorbent assay (ELISA). The analysis of each cytokine was performed according to the manufacturer's manual, and the absorbance was determined at 450nm by ELISA leader (VersaMax, Molecular Devices, USA).

As shown in Fig. 13, in COPD induced group, the level of IL-6 was sharply increased in BALF. The drug control group treated with Roflumilast showed significant reduction in the level of IL-6 compared with COPD induced group and the groups treated with 15 mg/kg and 30 mg/kg of KS534 showed reduced level of IL-6 by 35.8 % and 50.6 % comparing with COPD induced group. The level of TNF-alpha in BALF was sharply increased in COPD induced group. The drug control group treated with Roflumilast showed significant reduction in the level of TNF-alpha compared with COPD induced group and the groups treated with 15 mg/kg and 30 mg/kg of KS534 showed reduced level of TNF-alpha by 18.3 % and 27.6 % comparing with COPD induced group.

As shown in Fig. 14, the invasion of the inflamed cells was significantly increased in bronchiolar surroundings and hyperplasia of epithelial cells as well as hypertrophy of tracheal muscle were also found in COPD induced group whereas the invasion of the inflamed cells was significantly reduced in the positive control group treated with Roflumilast as well as the test sample group (30 mg/kg, KS534) orally administrated with various concentrations of test samples (KS 534).

In summary, the level of IL-6 and TNF-alpha in BALF as well as the number of inflammatory cells were remarkably increased in COPD-induced group and the reproduction of ROS and activity of elastase were sharply increased in COPD-induced group. In a while, the inventive KS534 compound effectively reduced the number of inflammatory mediator such as neutrophils and inflammatory cytokines such as IL-6 and TNF-alpha, which has been confirmed by histopathological analysis on broncho-alveolar tissue.

In conclusion, it has been confirmed that the inventive KS534 compound effectively inhibited the most of pathological syndromes of COPD comparing with the conventionally available drug (Roflumilast) used as a positive control group which did not show significant reduction on elastase activity. Accordingly, the inventive KS534 compound can be useful as a potent natural treating agent of COPD.

6-7. statistics

All the result obtained from various experiments was determined using by one-way ANOVA test and the statistical significance between respective group was verified according to Dunnett's multiple comparison test for post hoc comparison result.

**Experimental Example 7. Acute toxicity test of oral administration in rat**

The acute toxicity test was performed by administrating inventive compound to 6-weeks aged SPF Sprague-Dawley rats.

250 mg/kg, 500 mg/kg, 1000 mg/kg, 5000 mg/kg of inventive compounds was orally administrated to each group consisting of 2 rats and the symptoms of rats were observed for 14 days. After administrating the extract or compounds, all the clinical changes i.e., mortality, clinical signs, body weight changes was observed and blood test such as haematological test and hematological biochemistry test was performed. The abnormal changes of abdominal organ and thoracic organ were observed after autopsy.

There did not show any changes in mortality, clinical signs, body weight changes and gross findings in any group or either gender. Furthermore, there showed any toxicity in test group treated with 5000 mg/kg of inventive extract or compounds.

Accordingly, it has been confirmed that the inventive compounds prepared in the present invention was potent and safe substance showing $LD_{50}$ (more than 5000 mg/kg) in oral administration.

**Mode for the Invention**

Hereinafter, the formulating methods and kinds of excipients will be described, but the present invention is not limited to them. The representative preparation examples were described as follows.

Preparation of injection

KS534 ----- 100 mg
Sodium metabisulfite ----- 3.0 mg
Methyl paraben ----- 0.8 mg
Propyl paraben ----- 0.1 mg
Distilled water for injection ----- optimum amount

Injection preparation was prepared by dissolving active component, controlling pH to about 7.5 and then filling all the components in 2 mL ample and sterilizing by conventional injection preparation method.

Preparation of powder

KS534 ----- 500 mg
Corn Starch ----- 100 mg
Lactose ----- 100 mg
Talc ----- 10 mg

Powder preparation was prepared by mixing above components and filling sealed package.

Preparation of tablet

KS534 ----- 200 mg
Corn Starch ----- 100 mg
Lactose ----- 100 mg
Magnesium stearate ----- optimum amount

Tablet preparation was prepared by mixing above components and entabletting.

Preparation of capsule

KS534 ----- 100 mg
Lactose ----- 50 mg
Corn starch ----- 50 mg
Talc ----- 2 mg
Magnesium stearate ----- optimum amount

Tablet preparation was prepared by mixing above components and filling gelatin capsule by conventional gelatin preparation method.

Preparation of liquid

KS534 ----- 1000 mg
Sugar ----- 20 g
Polysaccharide ----- 20 g
Lemon flavor ----- 20 g

Liquid preparation was prepared by dissolving active component, and then filling all the components in 1000 mL ample and sterilizing by conventional liquid preparation method.

Preparation of health food

KS534 ----- 1000 mg
Vitamin mixture ----- optimum amount
Vitamin A acetate ----- 70 g
Vitamin E ----- 1.0 mg
Vitamin $B_{10.}$------ 13 mg
Vitamin $B_2$ ----- 0.15 mg
Vitamin $B_6$ ----- 0.5 mg
Vitamin $B_1$ ----- 20.2g
Vitamin C ----- 10 mg
Biotin ----- 10 g
Amide nicotinic acid ----- 1.7 mg
Folic acid ----- 50 g
Calcium pantothenic acid ----- 0.5 mg
Mineral mixture ----- optimum amount
Ferrous sulfate ----- 1.75 mg
Zinc oxide ----- 0.82 mg
Magnesium carbonate ----- 25.3 mg
Monopotassium phosphate ----- 15 mg

Dicalcium phosphate ----- 55 mg
Potassium citrate ----- 90 mg
Calcium carbonate ----- 100 mg
Magnesium chloride ----- 24.8 mg

The above mentioned vitamin and mineral mixture may be varied in many ways. Such variations are not to be regarded as a departure from the spirit and scope of the present invention.

Preparation of health beverage

KS534 ----- 1000 mg
Citric acid ----- 1000 mg
Oligosaccharide ----- 100 g
Apricot concentration ----- 2 g
Taurine ----- 1 g
Distilled water ----- 900 mL

Health beverage preparation was prepared by dissolving active component, mixing, stirred at 85°C for 1 hour, filtered and then filling all the components in 1000 mL ample and sterilizing by conventional health beverage preparation method.

The invention being thus described, it will be obvious that the same may be varied in many ways. Such variations are not to be regarded as a departure from the spirit and scope of the present invention, and all such modifications as would be obvious to one skilled in the art are intended to be included within the scope of the following claims.

**Industrial Applicability**

As described in the present invention, inventive KS534 compound from the extract of *Pseudolysimachion* rotundum var subintegrum showed potent anti-inflammatory, anti-allergy, anti-asthma and anti-COPD activity confirmed by through various in vitro tests, for example, (1) cyto-toxicity test using by HT1080, H292 and EL4 cell line, (2) an inhibition test on the expression of MUC5AC (oligomeric muscus/gel-forming) induced by TNF-alpha, (3) NF-kappa B luciferase reporter assay, (4) inhibition test on the activity of NF-kappa B transcription factor; (5) inhibition on mRNA expression of target genes such as MMP-9, MUC5AC and IL-4 by way of inhibiting the NF-kappa B activity; (6) dose-dependent inhibiting effect on MUC5AC reproduction through MUC5AC protein reproduction assay; as well as in vivo tests, for example, (7) reducing effect on the number of inflammatory cells such as eosinophils using by OVA-sensitized/challenged mouse model, (8) inhibition test on the release of IgE, inflammatory cytokines such as IL-4, IL-5, IL-13 etc in BALF fluid and invasion of inflamed cells, as well as the suppression of airway hyperresponsiveness and golblet cell hyperplasia, (9) an inhibition test using by COPD animal model (C57B/6N mouse) on the proliferation of inflammatory cells in BALF fluid, the inhibition on the reproduction of ROS (Reactive oxygen species), and activity of neutrophil elastase, the reducing effect on the level of IL-6, TNF-alpha and the infiltrated inflammatory cells etc. Therefore, it can be used as the therapeutics or functional health food for treating and preventing allergic disease, inflammatory disease, asthma or chronic obstructive pulmonary disease (COPD).

## Claims

1. (3R,5S,5aS,6R,7S,8R,8aS)-8-chloro-8a-hydroxy-5-(((2S,3R,4S,5S,6R)-3,4,5-trihydroxy-6-(hydroxymethyl)tetrahydro-2H-pyran-2-yl)oxy)-1H-3,6-methanocyclopenta[e][1,3]dioxepin-7-yl 3,4-dihydroxybenzoate (KS534) represented by the following chemical formula (1), a pharmaceutically acceptable salt or solvates thereof:

[Chemical Formula 1]

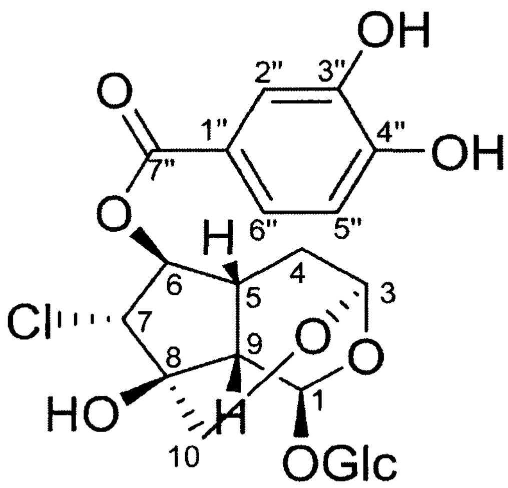

(1)

2. The compound of claim 1 (KS534), the pharmaceutically acceptable salt or solvates thereof for use in a method of treating or preventing allergic disease, inflammatory disease, asthma or chronic obstructive pulmonary disease (COPD) in mammals, wherein the method comprises administering a therapeutically effective amount of said compound (KS534), said pharmaceutically acceptable salt or solvates thereof into the mammal suffering from allergic disease, inflammatory disease, asthma or chronic obstructive pulmonary disease (COPD).

3. A pharmaceutical composition comprising the compound KS534, the pharmaceutically acceptable salt or solvates thereof as set forth in claim 1 isolated from *Pseudolysimachion rotundum* var *subintegrum,* and further comprising pharmaceutically acceptable carriers or excipients.

4. A pharmaceutical composition of claim 3 for use in a method of treating or preventing an allergic disease, inflammatory disease, asthma or chronic obstructive pulmonary disease (COPD).

5. A health functional food comprising a therapeutically effective amount of the compound (KS534), the pharmaceutically acceptable salt or solvates thereof as set forth in claim 1 as an active ingredients for the prevention or alleviation of allergic disease, inflammatory disease, asthma or chronic obstructive pulmonary disease (COPD).

## Patentansprüche

1. (3R,5S,5aS,6R,7S,8R,8aS)-8-Chlor-8a-hydroxy-5-(((2S,3R,4S,5S,6R)-3,4,5-trihydroxy-6-(hydroxymethyl)tetrahydro-2H-pyran-2-yl)oxy)-1H-3,6-methanocyclopenta[e][1,3]dioxepin-7-yl-3,4-dihydroxybenzoat (KS534), dargestellt durch die folgende chemische Formel (1), ein pharmazeutisch annehmbares Salz oder Solvate davon:

[Chemische Formel 1]

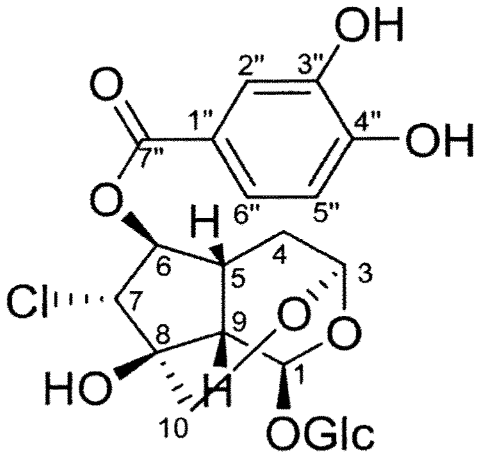

(1)

2. Verbindung nach Anspruch 1 (KS534), das pharmazeutisch annehmbare Salz oder Solvate davon zur Verwendung in einem Verfahren zum Behandeln oder Verhindern einer allergischen Erkrankung, einer entzündlichen Erkrankung, Asthma oder einer chronisch obstruktiven Lungenerkrankung (COPD) bei Säugern, wobei es das Verfahren umfasst, dem Säuger, der an einer allergischen Erkrankung, einer entzündlichen Erkrankung, Asthma oder einer chronisch obstruktiven Lungenerkrankung (COPD) leidet, eine therapeutisch wirksame Menge der Verbindung (KS534), des pharmazeutisch annehmbaren Salzes oder von Solvaten davon zu verabreichen.

3. Pharmazeutische Zusammensetzung, umfassend die Verbindung KS534, das pharmazeutisch annehmbare Salz oder Solvate davon, wie in Anspruch 1 dargelegt, die aus *Pseudolysimachion rotundum* var *subintegrum* isoliert sind, und weiterhin pharmazeutisch annehmbare Träger und Hilfsstoffe umfassend.

4. Pharmazeutische Zusammensetzung nach Anspruch 3 zur Verwendung in einem Verfahren zum Behandeln oder Verhindern einer allergischen Erkrankung, einer entzündlichen Erkrankung, Asthma oder einer chronisch obstruktiven Lungenerkrankung (COPD).

5. Funktionelles Gesundheitsnahrungsmittel, umfassend eine therapeutisch wirksame Menge der Verbindung (KS534), des pharmazeutisch annehmbaren Salzes oder von Solvaten davon, wie in Anspruch 1 dargelegt, als Wirkstoff zur Vorbeugung oder Linderung einer allergischen Erkrankung, einer entzündlichen Erkrankung, Asthma oder einer chronisch obstruktiven Lungenerkrankung (COPD).

**Revendications**

1. 3,4-Dihydroxybenzoate de (3R,5S,5aS,6R,7S,8R,8aS)-8-chloro-8a-hydroxy-5-(((2S,3R,4S,5S,6R)-3,4,5-trihydroxy-6-(hydroxyméthyl)tétrahydro-2H-pyran-2-yl)oxy)-1H-3,6-méthanocyclopenta[e][1,3]dioxépin-7-yle (KS534) représenté par la formule chimique (1) suivante, sel ou solvates pharmaceutiquement acceptables de celui-ci :

[Formule chimique 1]

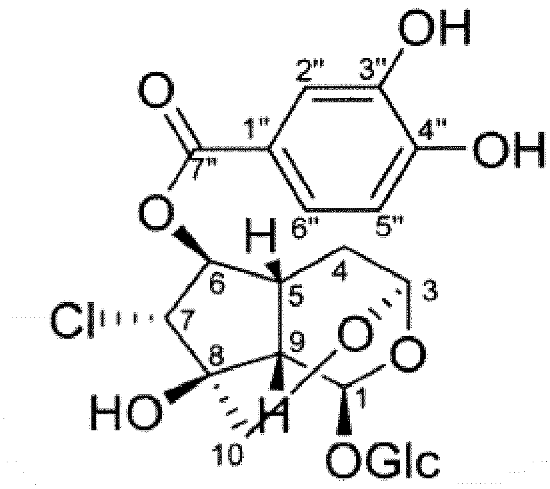

(1)

**2.** Composé selon la revendication 1 (KS534), sel ou solvates pharmaceutiquement acceptables de celui-ci destinés à être utilisés dans un procédé de traitement ou de prévention d'une maladie allergique, d'une maladie inflammatoire, de l'asthme ou d'une bronchopneumopathie chronique obstructive (BPCO) chez des mammifères, le procédé comprenant l'administration d'une quantité efficace sur le plan thérapeutique dudit composé (KS534), dudit sel ou desdits solvates pharmaceutiquement acceptables de celui-ci au mammifère souffrant de maladie allergique, de maladie inflammatoire, d'asthme ou de bronchopneumopathie chronique obstructive (BPCO).

**3.** Composition pharmaceutique comprenant le composé KS534, le sel ou les solvates pharmaceutiquement acceptables de celui-ci ainsi que présentés dans la revendication 1 isolés à partir de *Pseudolysimachion rotundum* var. *subintegrum,* et comprenant en outre des véhicules ou excipients pharmaceutiquement acceptables.

**4.** Composition pharmaceutique selon la revendication 3 destinée à être utilisée dans un procédé de traitement ou de prévention d'une maladie allergique, d'une maladie inflammatoire, de l'asthme ou d'une bronchopneumopathie chronique obstructive (BPCO).

**5.** Aliment à fonction de santé comprenant une quantité efficace sur le plan thérapeutique du composé (KS534), du sel ou des solvates de celui-ci ainsi que présentés dans la revendication 1 en tant que principes actifs pour la prévention ou le soulagement d'une maladie allergique, d'une maladie inflammatoire, de l'asthme ou d'une bronchopneumopathie chronique obstructive (BPCO).

[Fig. 1]

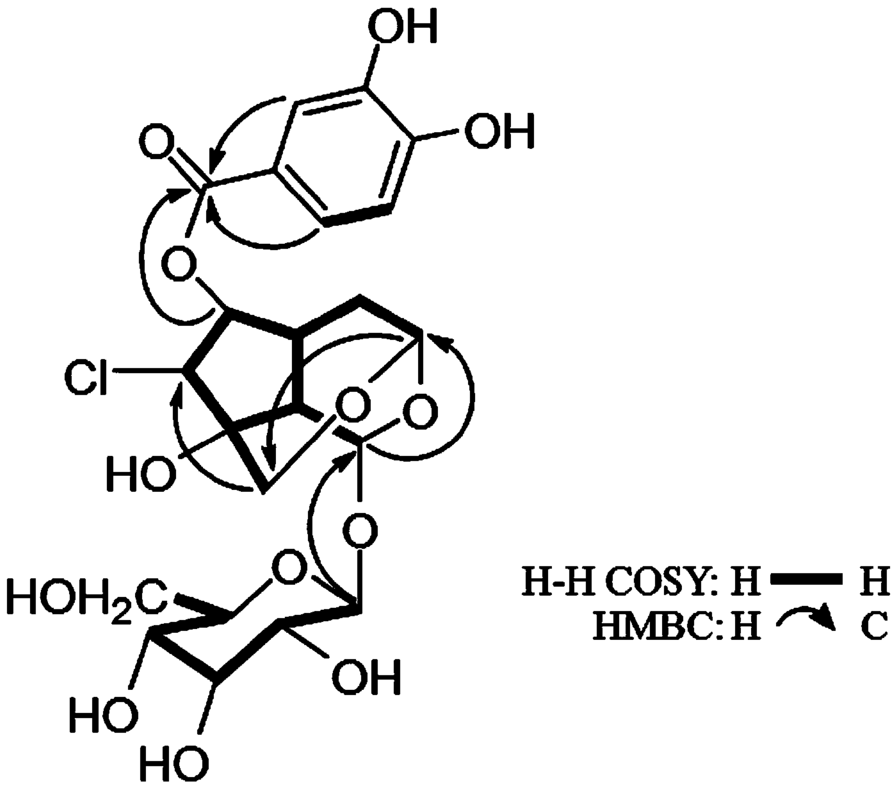

[Fig. 2]

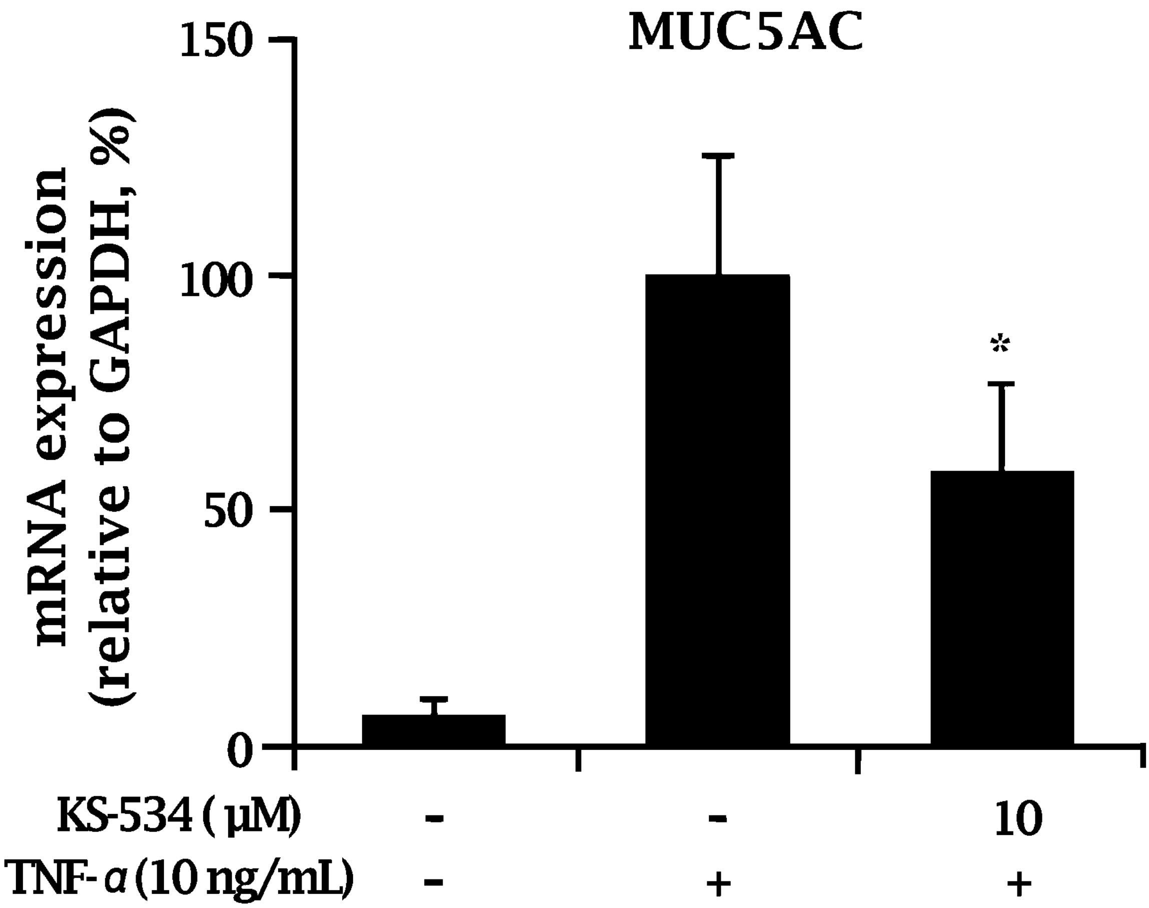
MUC5AC
mRNA expression
(relative to GAPDH, %)
150
100
50
0
*
KS-534 ( μM)
-
-
10
TNF-α (10 ng/mL)
-
+
+

[Fig. 3]

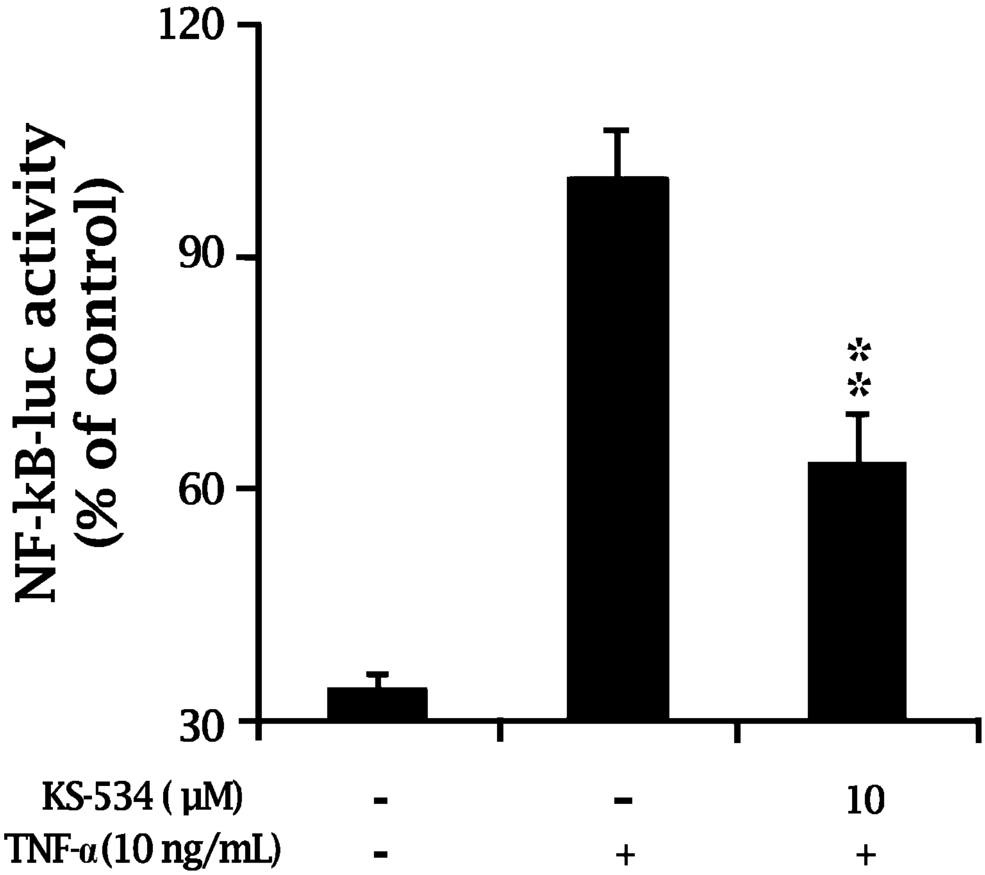
NF-kB-luc activity
(% of control)
120
90
60
30
**
KS-534 ( μM) - - 10
TNF-α(10 ng/mL) - + +

[Fig. 4]

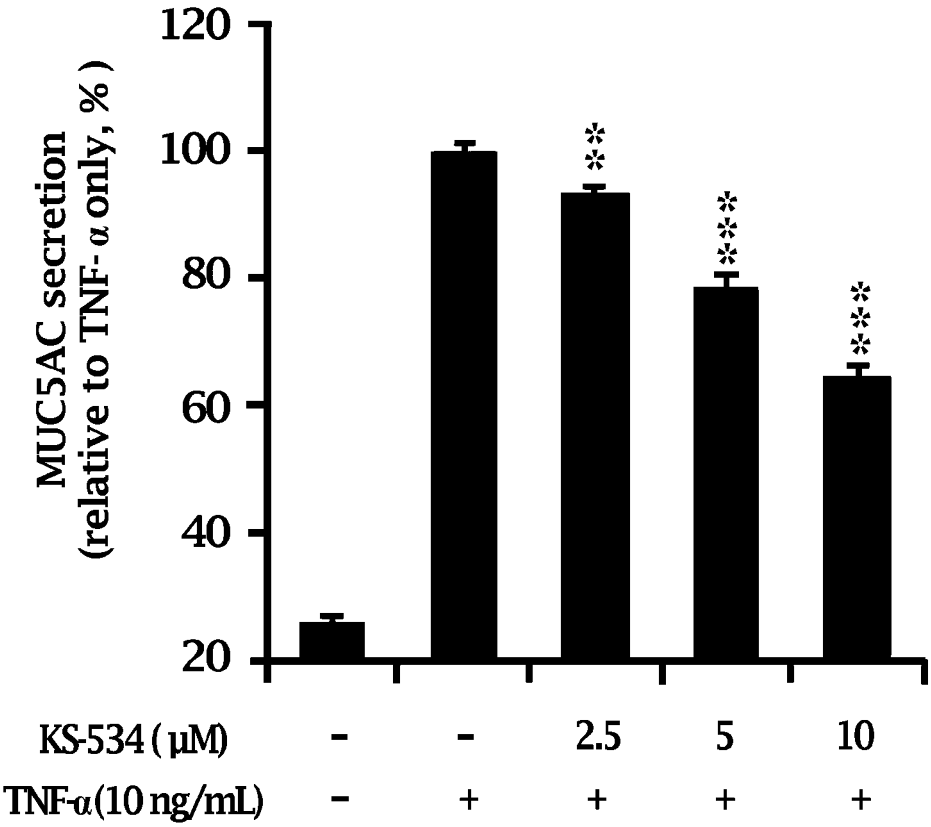
MUC5AC secretion
(relative to TNF-α only, %)
120
100
80
60
40
20
KS-534 (μM) - - 2.5 5 10
TNF-α(10 ng/mL) - + + + +

[Fig. 5]

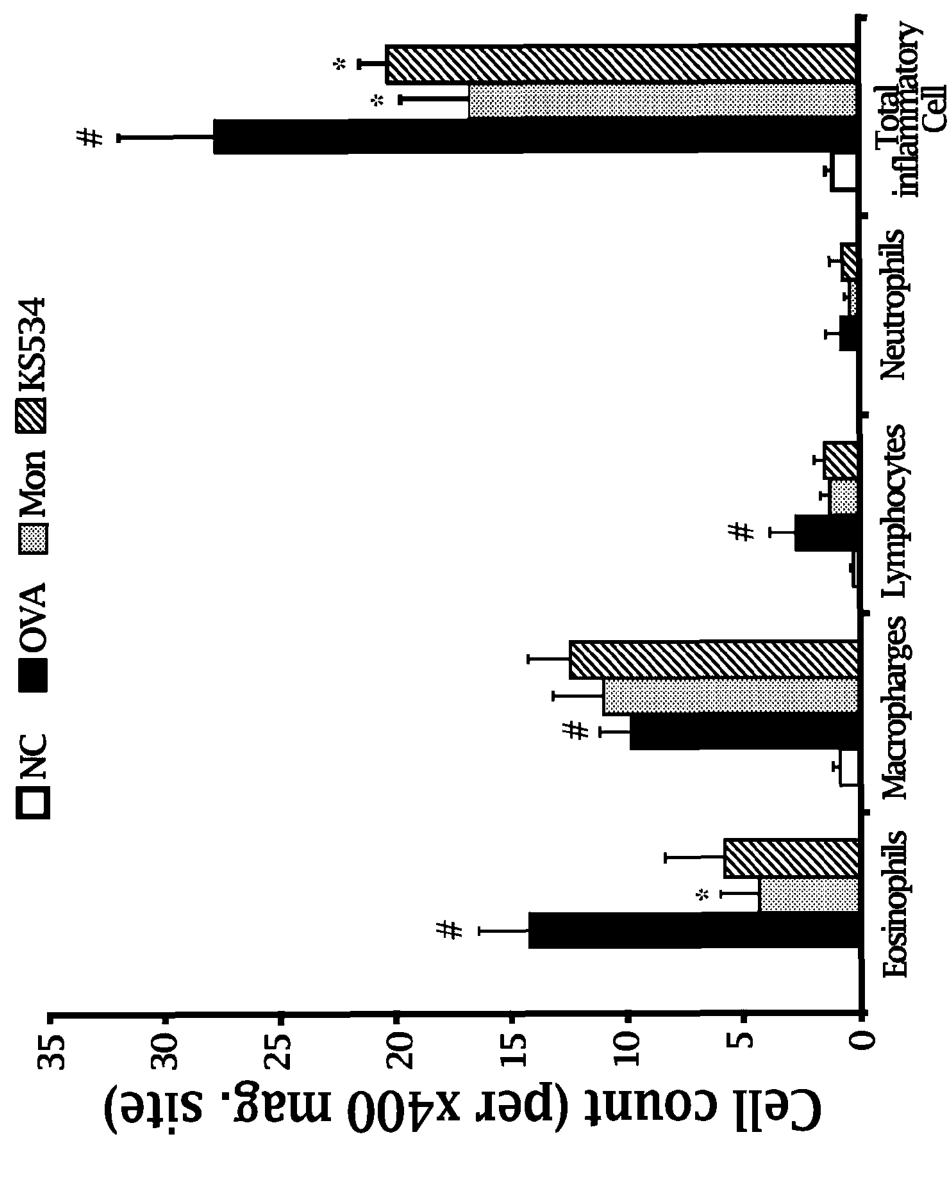
NC
OVA
Mon
KS534
Cell count (per x400 mag. site)
0
5
10
15
20
25
30
35
Eosinophils
Macropharges
Lymphocytes
Neutrophils
Total inflammatory Cell
#
*

[Fig. 6]

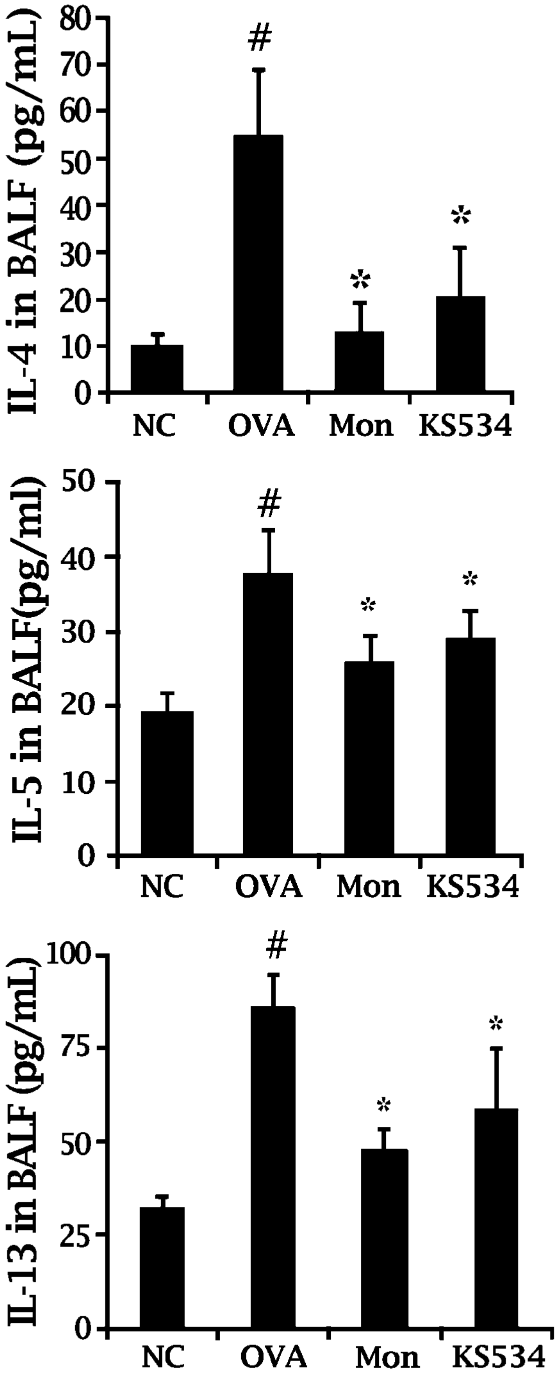
IL-4 in BALF (pg/mL)
80
70
60
50
40
30
20
10
0
#
*
*
NC
OVA
Mon
KS534
IL-5 in BALF(pg/ml)
50
40
30
20
10
0
#
*
*
NC
OVA
Mon
KS534
IL-13 in BALF (pg/mL)
100
75
50
25
0
#
*
*
NC
OVA
Mon
KS534

[Fig. 7]

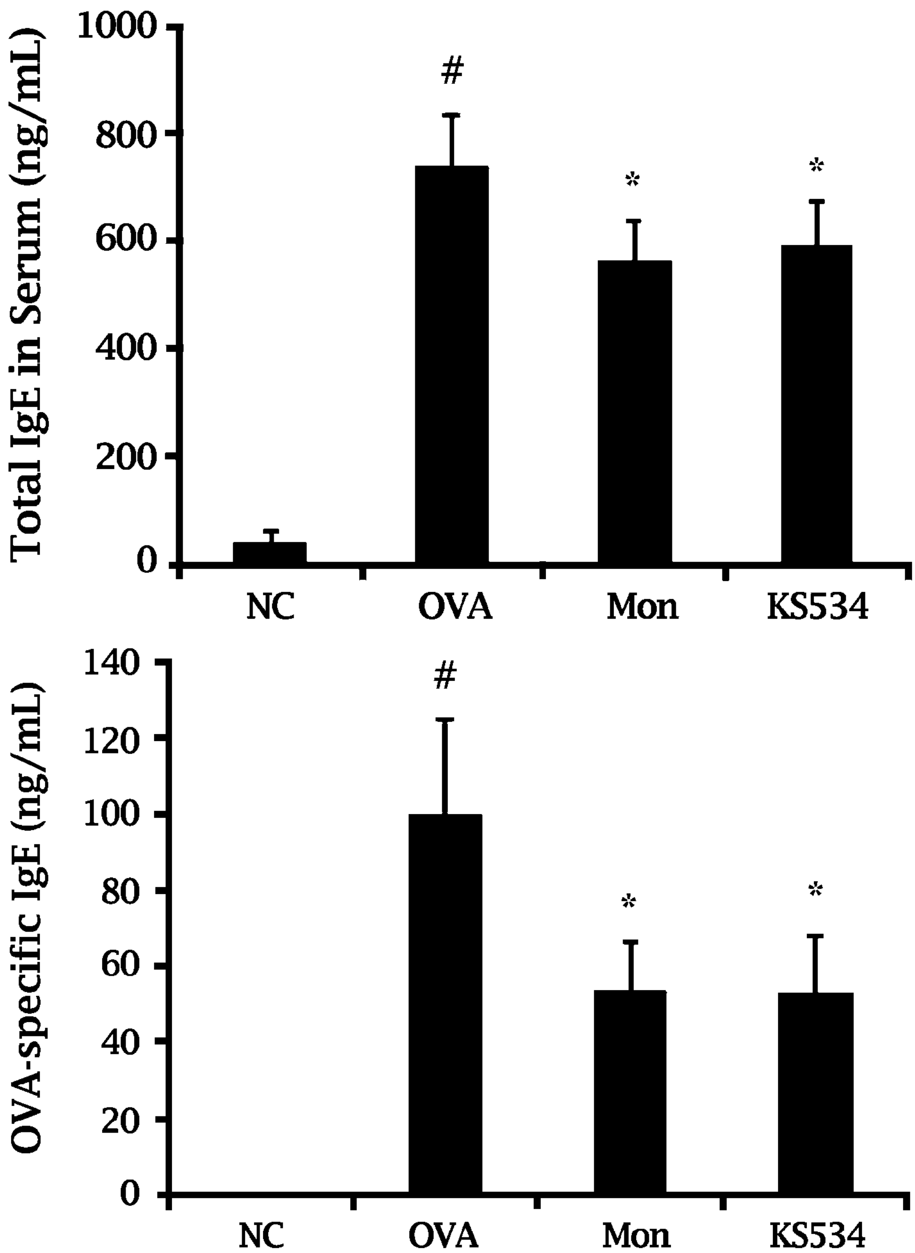
Total IgE in Serum (ng/mL)
1000
800
600
400
200
0
#
*
*
NC
OVA
Mon
KS534
OVA-specific IgE (ng/mL)
140
120
100
80
60
40
20
0
#
*
*
NC
OVA
Mon
KS534

[Fig. 8]

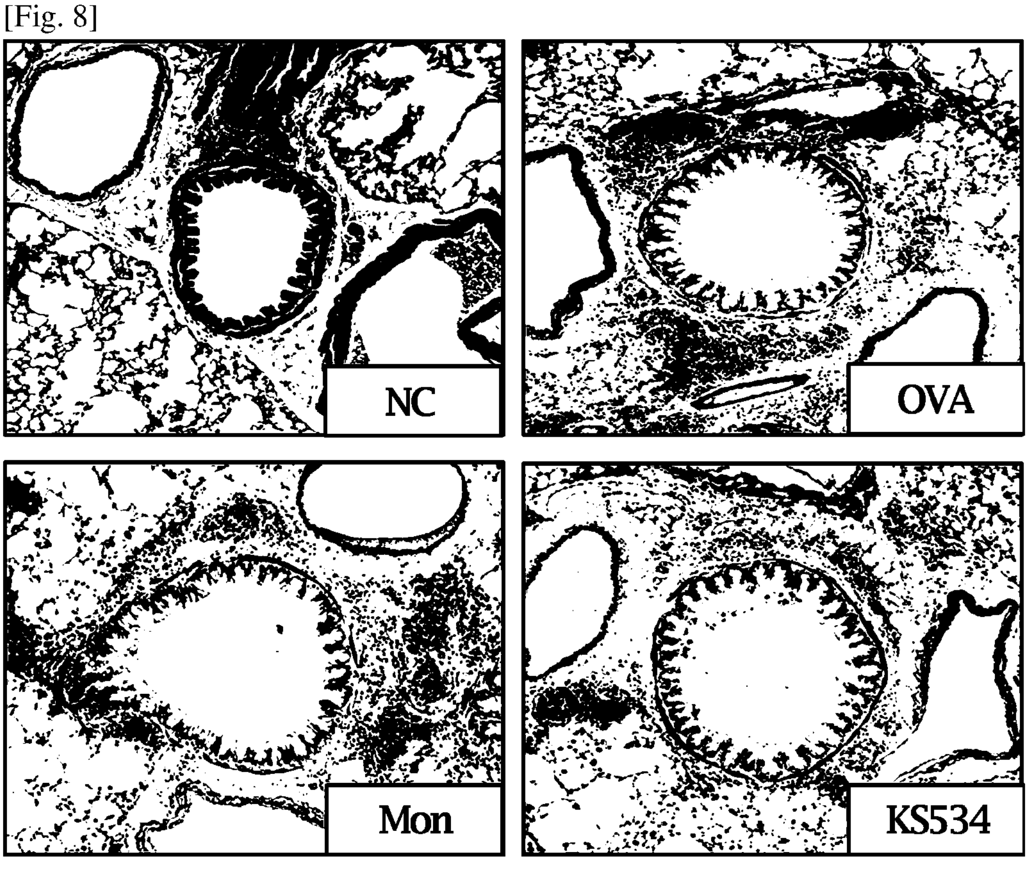

[Fig. 9]

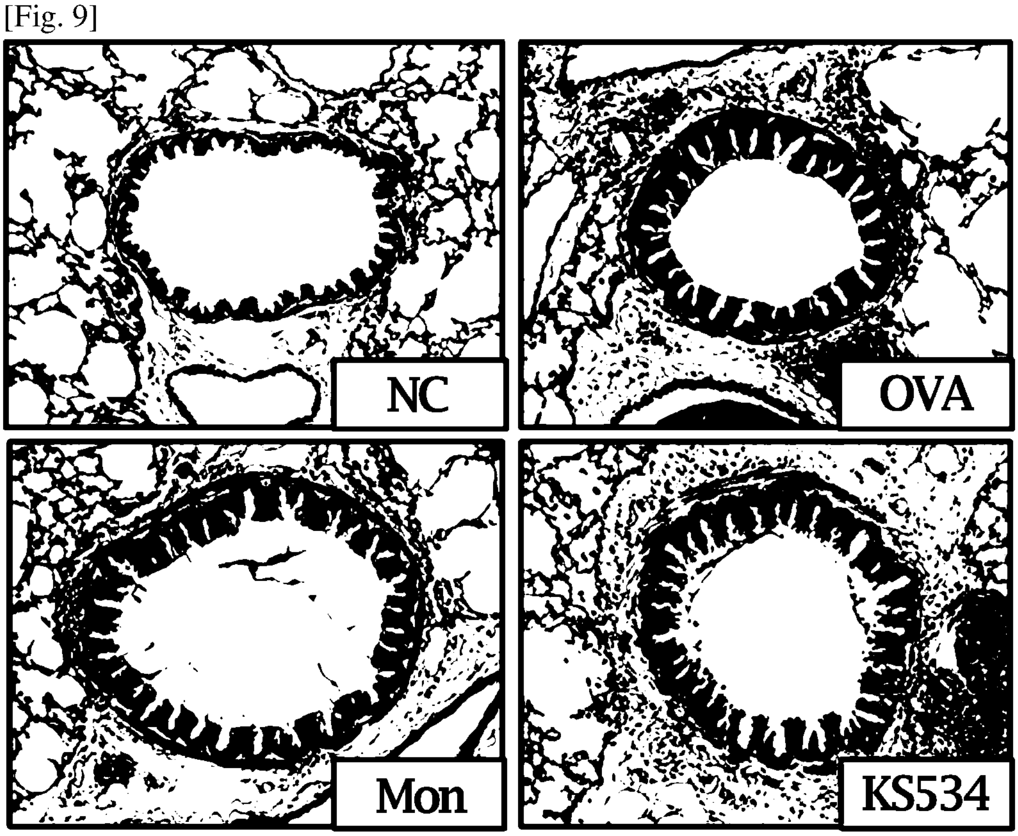

[Fig. 10]

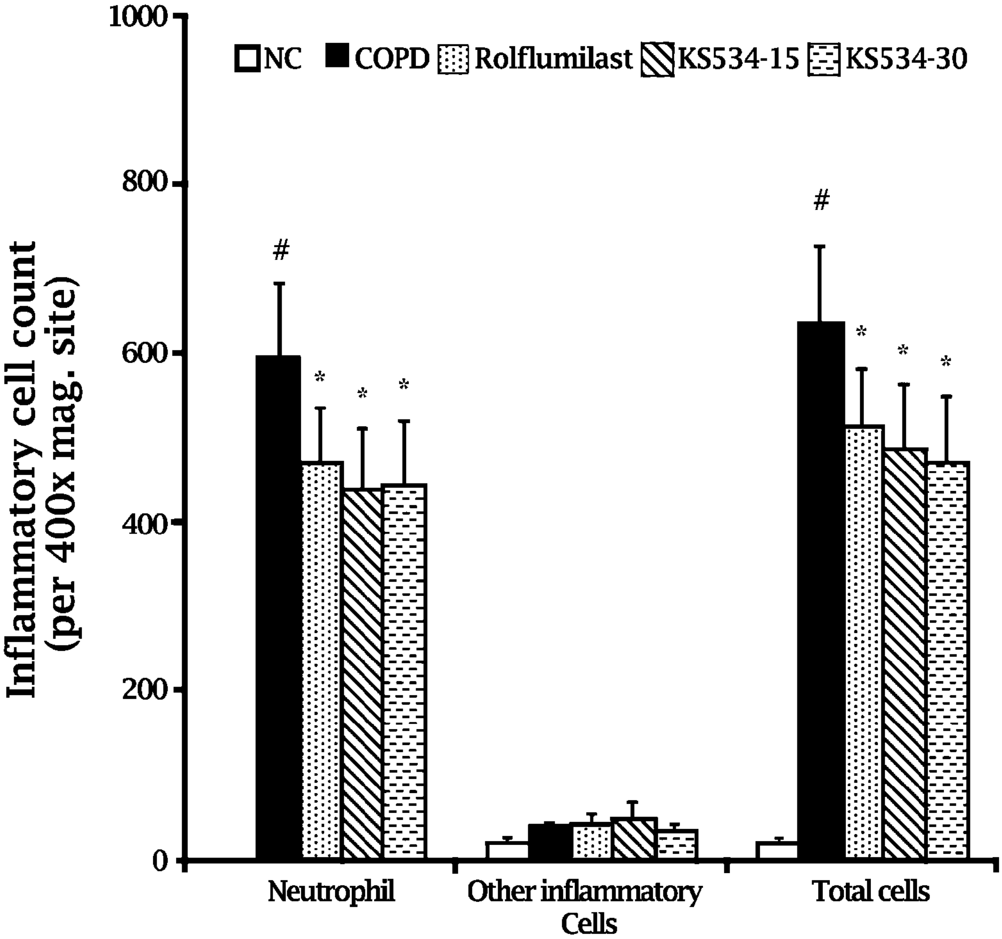
1000
800
600
400
200
0
NC
COPD
Rolflumilast
KS534-15
KS534-30
Inflammatory cell count
(per 400x mag. site)
#
*
Neutrophil
Other inflammatory
Cells
Total cells

[Fig. 11]

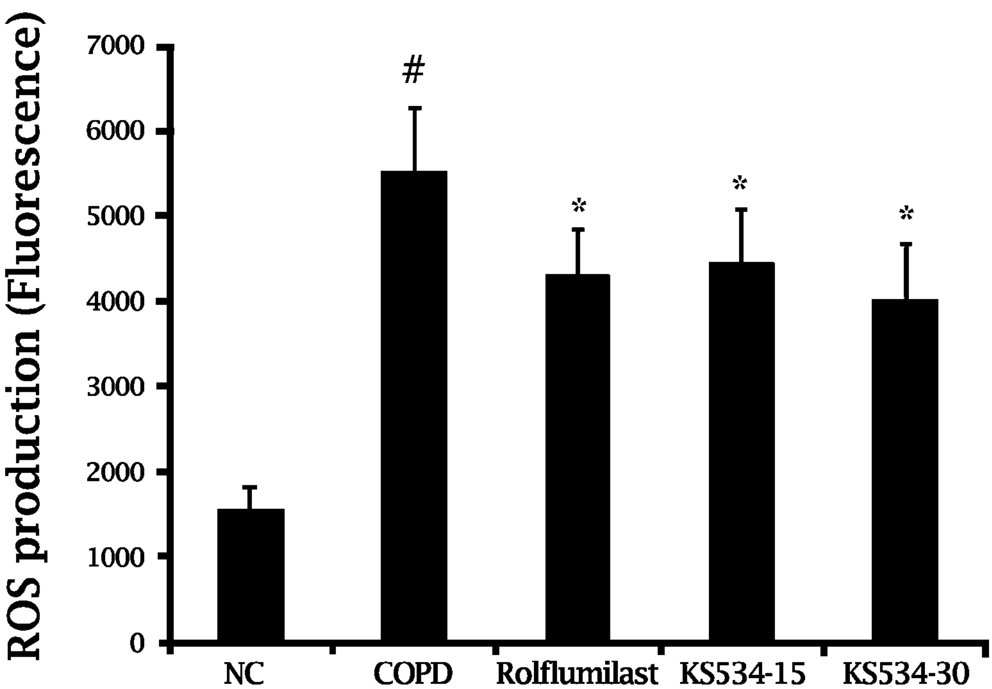
ROS production (Fluorescence)
7000
6000
5000
4000
3000
2000
1000
0
#
*
*
*
NC
COPD
Rolflumilast
KS534-15
KS534-30

[Fig. 12]

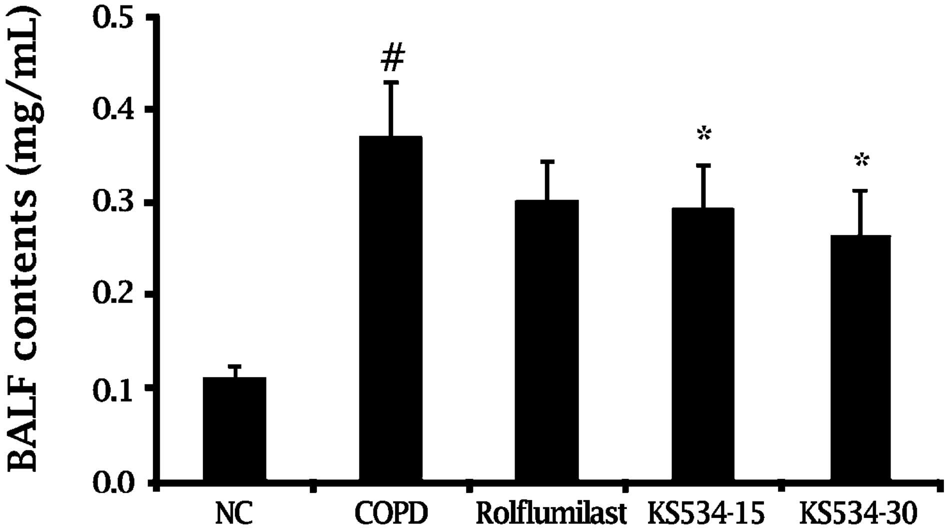
BALF contents (mg/mL)
0.5
0.4
0.3
0.2
0.1
0.0
#
*
*
NC
COPD
Rolflumilast
KS534-15
KS534-30

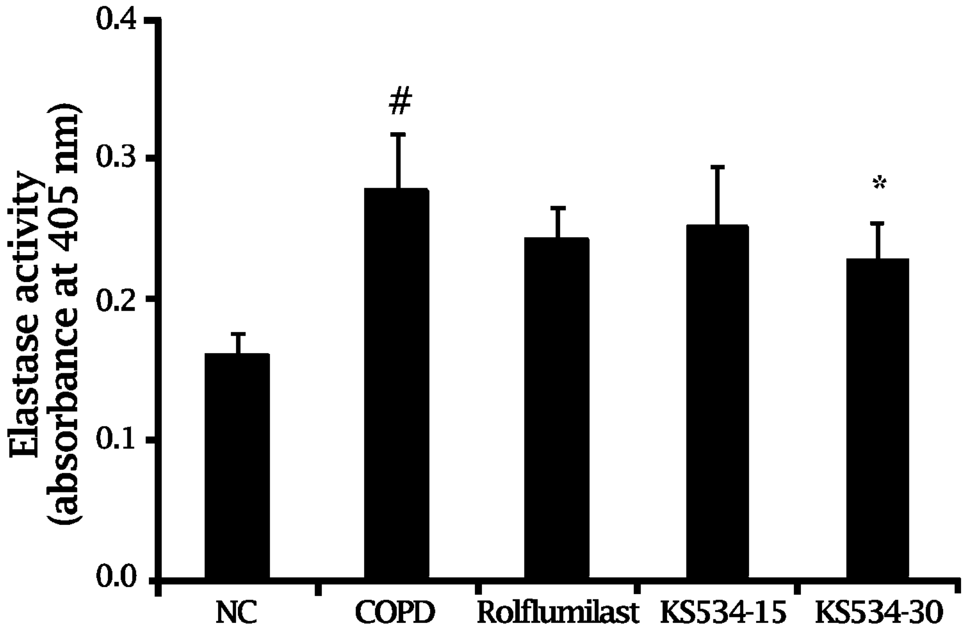
Elastase activity
(absorbance at 405 nm)
0.4
0.3
0.2
0.1
0.0
#
*
NC
COPD
Rolflumilast
KS534-15
KS534-30

[Fig. 13]

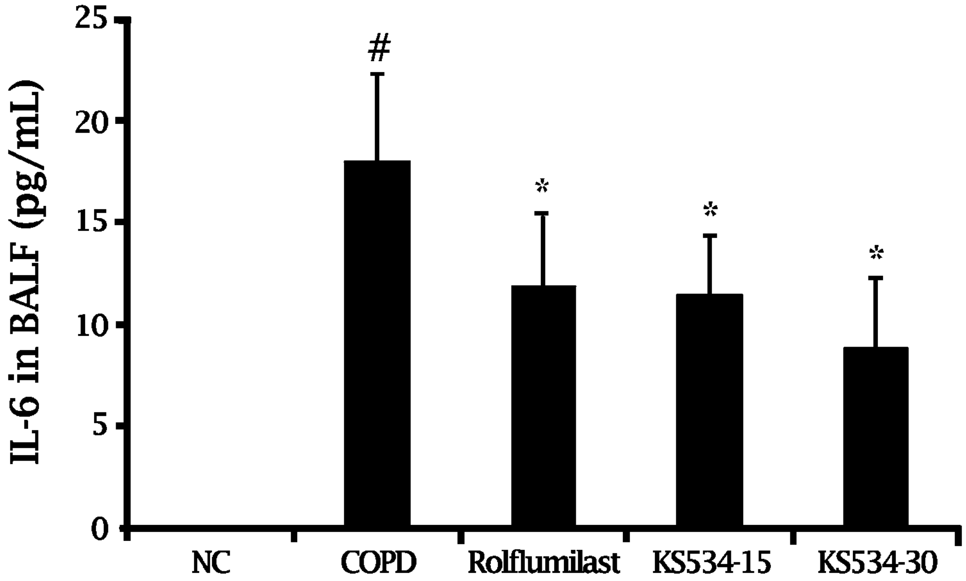
IL-6 in BALF (pg/mL)
25
20
15
10
5
0
#
*
*
*
NC
COPD
Rolflumilast
KS534-15
KS534-30

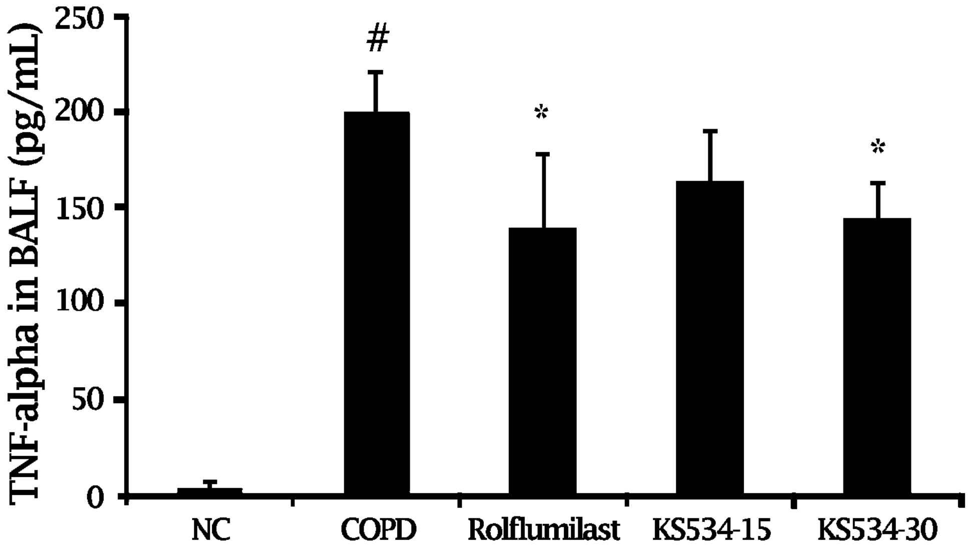
TNF-alpha in BALF (pg/mL)
250
200
150
100
50
0
#
*
*
NC
COPD
Rolflumilast
KS534-15
KS534-30

[Fig. 14]

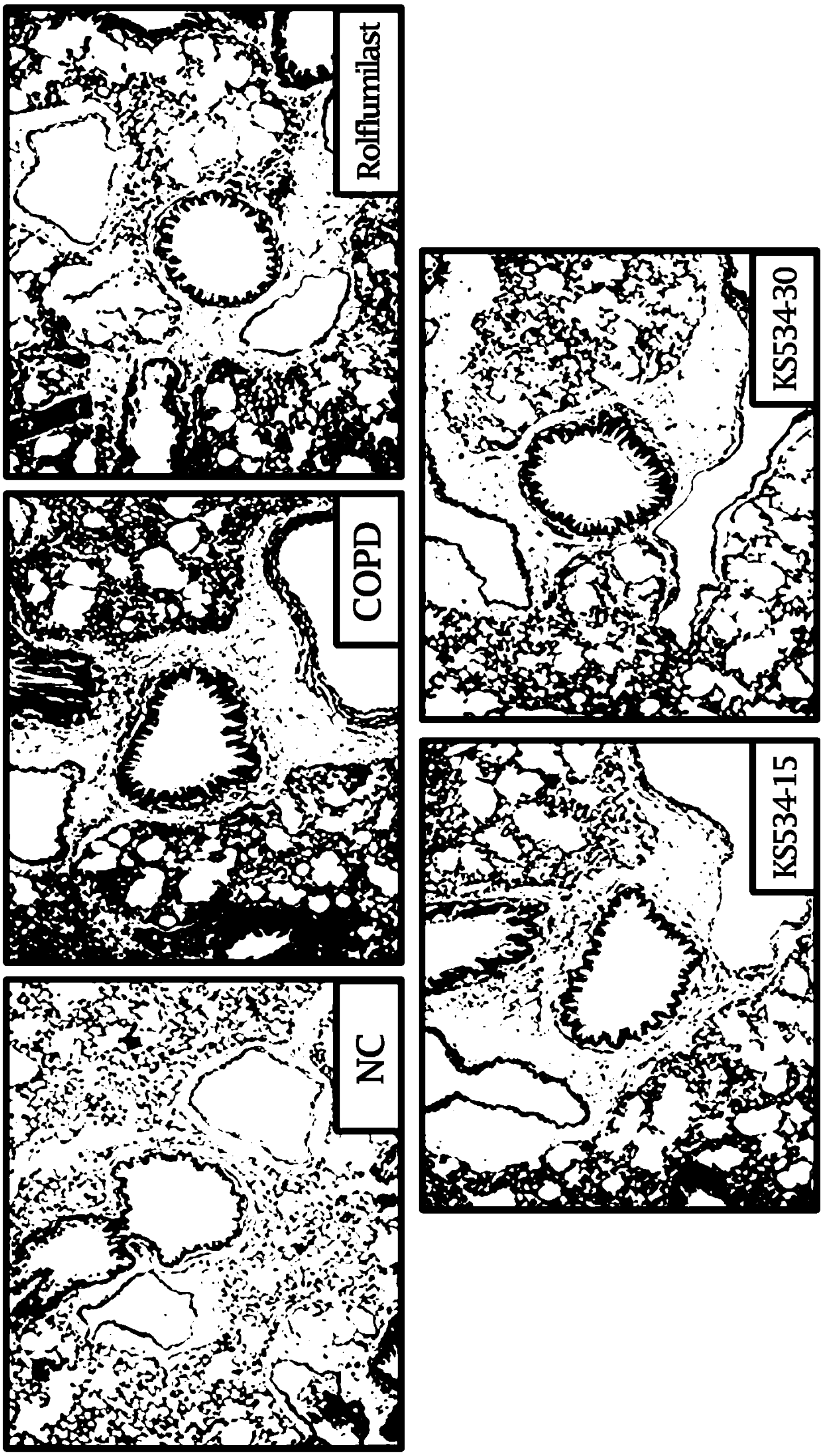

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 10860080B1
- US 20120183632 A1
- KR 102006125499 A1
- WO 2006129964 A1
- WO 2014104672 A1
- KR 1476045 B1
- KR 1504651 B1
- KR 1476095 B1
- KR 102006125499
- EP 110074 A
- WO 15824692 A
- KR 2015007647 W
- WO 1020140094023 A


### Non-patent literature cited in the description


- Pathophysiology of asthma. **MINOGUCHI K ; ADACHI M.** Rehabilitation of the patient with respiratory disease. McGraw-Hill, 1999, 97-104
- **ELIAS JA et al.** *J Clin Invest.,* 2003, vol. 111, 291-297
- **MAGGI E. et al.** *Immunotechnology,* 1998, vol. 3, 233-244
- **PAWANKAR R. et al.** *Curr. Opin. Allergy Clin. Immunol.,* 2001, vol. 1, 3-6
- **BARNES PJ et al.** *Phamacol. Rev.,* 1998, vol. 50, 515-596
- **BARNES P.J.** *Pharmacol.Rev.,* 2004, vol. 56, 515-548
- **KIM, D. S. et al.** *Am. J. Respir. Crit. Care Med.,* 2005, vol. 172, 842-847
- **SIN, D.D. et al.** *Proc. Am. Thorac. Soc.,* 2005, vol. 2, 8-11
- **BUIST A.S. et al.** *Lancet,* 2007, vol. 370, 741-750
- **BARNES PJ.** *Chest,* 2000, vol. 117, 10S-14S
- **SAETTA M. et al.** *Am. J. Respir. Crit. CareMed.,* 2001, vol. 163, 1304-1309
- **SAETTA M. et al.** *Am. J. Respir. Crit. Care Med.,* 1998, vol. 157, 822-826
- **DI STEFANO A. et al.** *Am. J. Respir. Crit. Care Med.,* 1998, vol. 158, 1277-1285
- **FABBRI L. et al.** *Thorax,* 1998, vol. 53, 803-808
- **FABBRI LM. et al.** *Am. J. Respir. Crit. Care Med.,* 2003, vol. 167, 418-424
- **HELE D. et al.** *Expert. Opino. Invest. Drug,* 2003, vol. 12, 5-18
- **FOX, J C. et al.** *Curr. Opin. Pharmacol.,* 2009, vol. 9, 231-242
- **ROWE ; RAYMOND C et al.** Handbook of Pharmaceutical Excipients. Pharmaceutical Press, 2012
- **SHIYAMA et al.** *Talanta,* 1997, vol. 44, 1299-1305
- **TOMINAGA et al.** *Anal. Commun.,* 1999, vol. 36, 47-50
- **KIM et al.** *J. Cell. Biochem.,* 2008, vol. 104, 1906-1917
- **PULIN CHE et al.** *Assay Drug Dev. Technol.,* 2012, vol. 10, 61-68
- **BUSSE, P. J. et al.** *J. Allergy Clin. Immun.,* 2005, vol. 116, 1256-1263
- **SMIRNOVA, M. G. et al.** *Cytokine,* 2000, vol. 12, 1732-1736
- **SIKDER, MA. et al.** *Phytother. Res.,* 2014, vol. 28, 62-68
- **SIKDER, M. A. et al.** *Phytother. Res.,* 2014, vol. 28, 62-68
- **TAKEYAMA, K. et al.** *Proc. Natl. Acad. Sci. U.S.A.,* 1999, vol. 96, 3081-3086
- **SHIN, I. S. et al.** *Food Chem. Toxicol.,* 2013, vol. 62, 506-513
- **KAY, A.B.** *N. Engl. J. Med.,* 2001, vol. 344, 30-37
- **KWAK YG. et al.** *J. Clin. Invest.,* 2003, vol. 111, 1083-1092
- **BHAVSAR, T. et al.** *J. Chronic Obstr. Pulm. Dis.,* 2008, vol. 3, 477-481